# EUROPEAN PATENT APPLICATION

(11) **EP 3 834 790 A1**
(43) Date of publication of application: **16.06.2021**
(21) Application number: 19847696.2
(22) Date of filing: 08.08.2019
(51) Int. Cl.: A61F 13/534, A61F 13/53, A61F 13/537

(54) **ABSORBENT ARTICLE**

(30) Priority: 09.08.2018 US 201862716410 P; 20.08.2018 US 201862719739 P; 29.08.2018 US 201862724105 P; 10.09.2018 US 201862728929 P; 17.09.2018 US 201862732031 P; 09.10.2018 US 201862742987 P; 07.11.2018 US 201862756662 P; 23.01.2019 US 201962795651 P; 25.01.2019 JP 2019011648
(71) Applicant: Yamada, Kikuo, Shinagawa-ku Tokyo 141-0022 (JP)
(72) Inventor: Yamada, Kikuo, Shinagawa-ku Tokyo 141-0022 (JP)
(74) Representative: Ishiguro, Masaoki
(86) International application number: PCT/JP2019/031442
(87) International publication number: WO 2020/032187

(57) **Abstract**

In order to realize an easy-to-use absorbent article having excellent absorbency of body fluids and having excellent usability, the absorbent article includes: a first absorbent body (4) configured to absorb body fluids; a second absorbent body (6) configured to absorb body fluids; and a thermally-fusible fiber (5) provided between the first absorbent body and the second absorbent body and capable of retaining the first absorbent body and the second absorbent body.

## Description

### Technical Field

The present invention relates to an absorbent article used in diapers, napkins, incontinence products, absorbent products for food, and the like.

### Background Art

There have been many proposals that, in absorbent articles used for diapers, napkins, incontinence products, and the like, an absorbent body provided with a super absorbent polymer (hereinafter, referred to as "SAP") can efficiently absorb body fluids such as urine and menstrual blood. As a method for fixing an SAP layer and a cover sheet of an absorbent body, a fixing method using hydrogen bonds or a fixing method using a thermally-fusible fiber has been proposed (Patent Literature 1).

### Citation List

### Patent Literature

Patent Literature 1: JP 2013-39804 A

### Summary of Invention

### Technical Problem

However, when the SAP layer is fixed to the cover sheet of the absorbent body, a graininess feeling (grainy feeling) of SAP is transmitted to the skin surface side. Thus, an unpleasant feeling is caused when a user wears the absorbent body. In addition, there has been room for improvement in the absorption performance of the absorbent body.

Therefore, an object of the present invention is to provide an easy-to-use absorbent article having excellent absorbency of body fluids and having excellent usability.

### Solution to Problem

The absorbent article according to claim 1 includes: a first absorbent body configured to absorb body fluids; a second absorbent body configured to absorb body fluids; and a thermally-fusible fiber provided between the first absorbent body and the second absorbent body and capable of retaining the first absorbent body and the second absorbent body.

The absorbent article according to claim 9 includes: an absorbent body configured to absorb body fluids; a thermally-fusible fiber capable of retaining the absorbent body; a cover sheet covering the absorbent body; and a flexible layer provided between the absorbent body and the cover sheet and having flexibility.

The absorbent article according to claim 16 includes: a first layer having a thermally-fusible fiber or a nonwoven fabric; a second layer having the thermally-fusible fiber or the nonwoven fabric; a first region in which an absorbent body is provided between the first layer and the second layer; and a second region in which the absorbent body is not provided between the first layer and the second layer, wherein the first layer and the second layer in the second region are melted and joined.

The absorbent article according to claim 20 includes: a first layer; a second layer; a first absorbent body provided between the first layer and the second layer, and provided in a state mixed with a fiber; a second absorbent body provided between the first layer and the second layer, and provided separately from the fiber.

In the absorbent article according to claim 23, a crushed pulp layer containing a thermally-fusible resin and a super absorbent polymer layer, that are stacked by an air flow, are joined to each other by heating with hot air and integrated.

The absorbent article according to claim 24 in which sides of at least one end and an opposing end of an absorbent body have a super absorbent polymer-absent region where a super absorbent polymer is substantially absent, and, in the portion, a flexibility layer and an absorbent body layer are joined by heating with hot air and integrated.

### Advantageous Effects of Invention

The invention according to claim 1 includes the thermally-fusible fiber capable of retaining the first absorbent body and the second absorbent body, so that the absorption performance of the absorbent body can be improved.

In the invention according to claim 9, the flexible layer is provided between the absorbent body and the cover sheet, so that it is possible to reduce a user's unpleasant feeling caused when the user wears the absorbent body.

In the invention according to claim 16, the second region in which the absorbent body is not provided is formed between the first layer and the second layer, and the first layer and the second layer in the second region are melted and joined, thereby reducing the risk of transfer of the absorbent body in the first region to the second region.

In the invention according to claim 20, the rigidity and softness of the absorbent article can be adjusted by the first absorbent body provided in a state mixed with a fiber; the second absorbent body separately from the fiber.

The invention according to claim 23 has less risk of degrading the absorption performance of the super absorbent polymer, and has a thin sheet thickness and excellent absorbency of body fluids, as compared to an absorbent article obtained by joining and integrating a crushed pulp layer and an absorbent body layer containing a super absorbent polymer using a hot-melt adhesive.

In the invention according to claim 24, it is possible to increase the proportion of the super absorbent polymer, so that it is possible to blend a large proportion of the super absorbent polymer and provide an absorbent article having a large absorption capacity.

### Brief Description of Drawings

Fig. 1 is a cross-sectional view of an absorbent article of a first embodiment.
Fig. 2 is a diagram illustrating a production line for a multi-ply sheet.
Fig. 3 is a cross-sectional view of an absorbent article of a second embodiment.
Fig. 4 is a cross-sectional view of an absorbent article of a third embodiment.
Fig. 5 is a diagram illustrating a production line for a multi-ply sheet.
Fig. 6 is a diagram illustrating a production line for a multi-ply sheet.
Figs. 7(a) to 7(c) are diagrams illustrating arrangement examples of an SAP supply device.
Fig. 8 is a cross-sectional view of an absorbent article of a fourth embodiment.
Fig. 9(a) to 9(c) are diagrams illustrating variations of a region without a first absorbent body and a second absorbent body.
Fig. 10 is a multi-ply sheet produced on the production line of Fig. 5.
Fig. 11 is a diagram illustrating a cross section A-A of the multi-ply sheet of Fig. 10.
Figs. 12(a) and 12(b) are diagrams illustrating outlines of the SAP supply device.
Fig. 13 is a diagram illustrating a masking member.
Fig. 14 is a diagram illustrating an example in which a second masking portion is used as a separate member.
Fig. 15 is a diagram illustrating an example in which a second masking portion is used as a separate member.
Fig. 16 is a diagram illustrating an example of an SAP-absent region.
Fig. 17 is a diagram showing a modified example of the SAP-absent region.
Fig. 18 is a cross-sectional view of an absorbent article of a fifth embodiment.
Fig. 19 is a diagram illustrating details of recess portions.
Fig. 20 is a diagram illustrating a production line for a multi-ply sheet.
Fig. 21 is a cross-sectional view of an absorbent article of a sixth embodiment.
Fig. 22 is a diagram illustrating a production line for a multi-ply sheet.
Fig. 23 is a cross-sectional view of an absorbent article of a seventh embodiment.
Fig. 24 is a diagram illustrating a production line for a multi-ply sheet.
Fig. 25 is a cross-sectional view of an absorbent article of an eighth embodiment.
Fig. 26 is a diagram illustrating a production line for a multi-ply sheet.
Fig. 27 is a cross-sectional view of an absorbent article of a ninth embodiment.
Fig. 28 is a diagram illustrating a production line for a multi-ply sheet.

### Description of Embodiments

The absorbent article of the present invention is described below in detail with reference to the drawings.

Note that the absorbent article of the present embodiment may be used for babies, adults, or children. Further, there is no limitation in terms of gender, and the absorbent article may be used for both men and women. Furthermore, the absorbent article may be used not only for humans, but also for pets such as dogs and cats.

In addition, the absorbent article of the present embodiment may be used not only for the purpose of absorbing urine, but also for the purpose of absorbing filth such as all other excrement or for the purpose absorbing menstrual blood for women, and may be used for the purpose of absorbent products for food (for example, trays or drip-protection sheets).

### First Embodiment

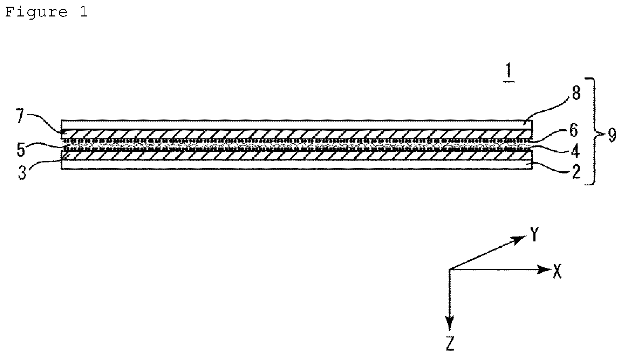

Fig. 1 is a cross-sectional view of an absorbent article 1 of a first embodiment, and a plurality of sheets extending in orthogonal X-Y planes is layered along a Z-direction orthogonal to the X-Y planes.

The absorbent article 1 is a multi-ply sheet 9 obtained by layering a first liquid-diffusible fiber sheet 2 as a cover sheet at the non-skin surface side of a wearer, a first pulp layer 3 as a first flexible layer, a first absorbent body 4, a thermally-fusible fiber (thermally adhesive fiber) 5, a second absorbent body 6, a second pulp layer 7 as a second flexible layer, and a second liquid-diffusible fiber sheet 8 as a cover sheet at the skin surface side.

Note that a hydrophilic surface sheet may be provided at the skin surface side of the second liquid-diffusible fiber sheet 8, and for example, the elastic surface sheet described in WO2017/150425 A may be used. Further, a moisture-permeable film may be provided as a liquid-impermeable sheet at the non-skin surface side of the first liquid-diffusible fiber sheet 2. Note that the basis weight of each sheet of the absorbent article 1 is set so that the thickness is in a range of about 2 mm to 6 mm.

### (First Liquid-Diffusible Fiber Sheet 2 and Second Liquid-Diffusible Fiber Sheet 8)

For the first liquid-diffusible fiber sheet 2 and the second liquid-diffusible fiber sheet 8 (hereinafter, the first liquid-diffusible fiber sheet 2 and the second liquid-diffusible fiber sheet 8 are collectively referred to as "liquid-diffusible fiber sheet"), a material containing pulp as a main raw material, i.e., a material containing a cellulose-based component can be used. Examples of the material containing a cellulose-based component include various paper materials such as thin paper (e.g., tissue paper and crepe paper), and various nonwoven fabrics such as spunbonded nonwoven fabrics. It is preferable to use a liquid-diffusible fiber sheet having a basis weight of 5 g/m² to 15 g/m². In the present embodiment, the first liquid-diffusible fiber sheet 2 and the second liquid-diffusible fiber sheet 8 are hydrophilic fiber sheets.

Further, when the paper material is used for the first liquid-diffusible fiber sheet 2 and the second liquid-diffusible fiber sheet 8, a pulp content is preferably 30% or more, and more preferably 80% or more. The second liquid-diffusible fiber sheet 8 is preferably embossed in order to impart flexibility and to provide fine pores for promoting the transfer of body fluids.
Similarly, the first liquid-diffusible fiber sheet 2 may be embossed. In this case, the shape of the embossing formed on the liquid-diffusible fiber sheet is not particularly limited, and any shape known in the related art may be formed by embossing. Further, the shape of the embossing formed on the liquid-diffusible fiber sheet may be always identical, or a plurality of different shapes may be used in combination. Note that the basis weight of the first liquid-diffusible fiber sheet 2 and the basis weight of the second liquid-diffusible fiber sheet 8 may be identical or different. As will be described below, the first liquid-diffusible fiber sheet 2 with the crushed pulp carried thereon is vacuum-sucked by an airflow forming device 105. In order to allow the airflow forming device 105 to vacuum-suck the crushed pulp, the first liquid-diffusible fiber sheet 2 preferably has an air permeability of 500 mm³/(mm² × sec) or more and 2000 mm³/(mm² × sec) or less. Note that in the present embodiment, the first liquid-diffusible fiber sheet 2 is the cover sheet on the side of the first absorbent body 4, and the second liquid-diffusible fiber sheet 8 is the cover sheet on the side of the second absorbent body 6.

Among the sheets constituting the absorbent article 1, a diffusion rate of liquids of the first liquid-diffusible fiber sheet 2 and the second liquid-diffusible fiber sheet 8 is faster than a diffusion rate of liquids of the first pulp layer 3 and the second pulp layer 7. Thus, the second liquid-diffusible fiber sheet 8 can diffuse body fluids and transfer the body fluids to the second absorbent body 6 through the second pulp layer 7. The first liquid-diffusible fiber sheet 2 can diffuse the body fluids that have not been able to be absorbed by the first absorbent body 4 and transfer the body fluids to the moisture-permeable film as the liquid-impermeable sheet as described above. Therefore, it is possible to reduce the reversion of the body fluids to the second liquid-diffusible fiber sheet 8, and it is possible to realize excellent rewetting properties.

In other words, since a plurality of liquid-diffusible fiber sheets is used in the absorbent article 1 of the first embodiment, the body fluids are diffused and quickly transferred to the non-skin surface side, and the reversion to the skin surface side is reduced.

Note that at least one of the first liquid-diffusible fiber sheet 2 or the second liquid-diffusible fiber sheet 8 may be constituted using various nonwoven fabrics such as spunbonded nonwoven fabrics, spunlaced nonwoven fabrics, thermally bonded nonwoven fabrics, air-through nonwoven fabrics, chemically bonded nonwoven fabrics, and melt blown nonwoven fabrics.

### (First Pulp Layer 3 and Second Pulp Layer 7)

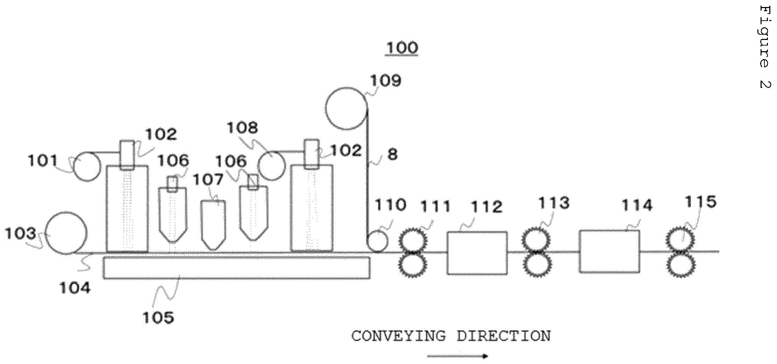

The first pulp layer 3 and the second pulp layer 7 can be constituted by a layer obtained by stacking pulp fibers obtained by crushing a pulp raw fabric 101 by a defibration device 102 as illustrated in Fig. 2 described below. When the first pulp layer 3 and the second pulp layer 7 are formed by stacking pulp fibers, flexibility can be imparted to the absorbent article 1. Thus, the user does not have an unpleasant feeling when wearing the absorbent article 1. Further, when the absorbent body has a super absorbent polymer (SAP), it is possible to reduce the risk that the user has a graininess feeling (roughness feeling) of SAP. Note that a space is formed between a plurality of pulp fibers, so it is possible to retain the SAP and the thermally-fusible fiber 5 in this space.

Further, the first pulp layer 3 and the second pulp layer 7 have liquid retainability (absorbency), because both the first and second pulp layers are produced by stacking pulp fibers. Thus, the second pulp layer 7 can retain body fluids from the second liquid-diffusible fiber sheet 8, so that it is possible to prevent a large amount of the body fluids from being transmitted to the second absorbent body 6. Therefore, it is possible to prevent inhibition of body fluid absorption due to gel blocking that occurs when the SAP absorbs body fluids.

Note that the amount of pulp fibers in the first pulp layer 3 may be set to be identical to the amount of the second pulp layer 7, and the amount of pulp fibers in the second pulp layer 7 at the skin surface side may be set to be larger than the amount of pulp fibers in the first pulp layer 3 at the non-skin surface side. Further, the first pulp layer 3 may be omitted.

### (First Absorbent Body 4 and Second Absorbent Body 6)

The first absorbent body 4 and the second absorbent body 6 preferably have the SAP as an absorbent material for absorbing body fluids. For the second absorbent body 6, it is preferable to use the SAP having a larger average particle size (volume) than that of the first absorbent body 4. The SAP having a large average particle size (volume) can absorb a large amount of body fluids, compared to the SAP having a small average particle size (volume). Meanwhile, the SAP having a small average particle size (volume) can be arranged in a larger amount than the SAP having a large average particle size (volume), and has a large surface area in contact with body fluids, so that the body fluids can be absorbed more quickly. Thus, the absorbent article 1 of the first embodiment can quickly transfer the body fluids to the non-skin surface side. The SAP of the first absorbent body 4 has an average particle size of 150 µm to 300 µm, the SAP of the second absorbent body 6 has an average particle size of 300 µm to 500 µm, and it is preferable to make the average particle size of the SAP of the second absorbent body 6 larger than the average particle size of the SAP of the first absorbent body 4. The first absorbent body 4 and the second absorbent body 6 may be constituted by only the SAP, or the SAP and crushed pulp (may be constituted by a mixture of both the SAP and crushed pulp, or an SAP layer and a crushed pulp layer). Alternatively, the first absorbent body and the second absorbent body may be constituted by only the crushed pulp, and preferably have the SAP.

For example, in the first absorbent body 4, the thermally-fusible fiber 5 may be melted to fix (retain) the SAP. In the first embodiment, 1 g to 5 g of the SAP having an average particle size of 150 µm to 300 µm is provided.

Further, in the second absorbent body 6, the thermally-fusible fiber 5 may be melted to fix (retain) the SAP. In the first embodiment, 1 g to 5 g of the SAP having an average particle size of 300 µm to 500 µm is provided.

The first absorbent body 4 and the second absorbent body 6 are illustrated as separate layers in Fig. 1. A part of the SAP of the first absorbent body 4 and a part of the SAP of the second absorbent body 6 are retained by the thermally-fusible fiber 5 to which the parts of the SAPs are fused in a state retained in a space formed between fibers constituting the first pulp layer 3 and the second pulp layer 7. Further, the part of the SAP of the first absorbent body 4 and the part of the SAP of the second absorbent body 6 are connected through the thermally-fusible fiber 5 to which the parts of the SAPs are fused. This makes it possible to reduce the transfer of the SAP of the first absorbent body 4 and the SAP of the second absorbent body 6 inside the absorbent article 1.

Here, regarding the absorbent article 1 for adults, the dimension in the horizontal direction (X-direction in Fig. 1) is 13 cm, and the dimension in the vertical direction (Y-direction in Fig. 1) is 45 cm. Assuming that 5 g of SAP for the first absorbent body 4 and 5 g of SAP for the second absorbent body 6 are provided in order to absorb about 300 cc of body fluids, the volume of SAP to be provided per unit area is about 85 g/m². Even when the absorbent article 1 for adults absorbs about 100 cc of body fluids, the volume of SAP to be provided is about 74 g/m².

The absorbent article 1 for babies has the dimension of the horizontal direction of about 12 cm and the dimension of the vertical direction of about 35 cm. Assuming that 2 g of SAP for the first absorbent body 4 and 2 g of SAP for the second absorbent body 6 are provided, the volume of SAP to be provided per unit area is about 48 g/m². Note that the above-mentioned dimensions are merely examples, and the dimensions may be changed as appropriate.

### (Thermally-Fusible Fiber 5)

As the thermally-fusible fiber 5, for example, a fiber configured to include a low melting point component and a high melting point component can be used. Particularly, it is preferable to use a core-sheath type or eccentric type thermally-welding composite fiber including polyethylene and polypropylene, and polyethylene and polyethylene terephthalate; and having a polyethylene layer with a low melting point provided at the surface side. In this case, the SAP is fixed and retained by melting the outer resin having a low melting point in a heating device 112 and a heating device 114 described below. Further, a part of the melted thermally-fusible fiber 5 is also mixed into the first pulp layer 3 and the second pulp layer 7, and a part of the first pulp layer 3 and at least a part of the SAP are connected through the fused thermally-fusible fiber 5. Similarly, a part of the second pulp layer 7 and at least a part of the SAP are connected through the fused thermally-fusible fiber 5. This makes it possible to reduce the transfer of the SAP of the first absorbent body 4 and the SAP of the second absorbent body 6 inside the absorbent article 1. As the thermally-fusible composite fiber, for example, ES fiber, manufactured by JNC Corporation, can be used.

Note that the thermally-fusible fiber 5, although illustrated as a separate layer in Fig. 1, enters at least one of the first pulp layer 3, the first absorbent body 4, the second absorbent body 6, and the second pulp layer 7 due to melting, and may be unrecognizable.

### (Multi-ply Sheet Production Method)

Fig. 2 is a diagram illustrating a production line 100 for the multi-ply sheet 9. The pulp raw fabric 101 is crushed into, for example, cotton-like shape by the defibration device 102 to form pulp fiber pieces (hereinafter referred to as "crushed pulp"). The defibration device 102 has a cutter, and the type and driving method of the cutter may be adjusted to regulate the roughness of the pulp to be crushed or the fiber length.

Meanwhile, the first liquid-diffusible fiber sheet 2 (not illustrated in Fig. 2) unwound from a liquid-diffusible fiber sheet raw fabric 103 is conveyed on a mesh-shaped conveyance belt 104 along the conveying direction (Y-direction in Figs. 1, 3, and 4). In a state where the first liquid-diffusible fiber sheet 2 is sucked by the airflow forming device 105, the crushed pulp is sucked by the airflow forming device 105 through the first liquid-diffusible fiber sheet 2. The airflow forming device 105 is connected to a vacuum source (not illustrated), and is configured such that the crushed pulp is stacked on the conveyance belt 104 by vacuum suction. The first pulp layer 3 is formed by stacking the crushed pulp on the conveyance belt 104 through the first liquid-diffusible fiber sheet 2 by vacuum suction of the vacuum source. Note that the airflow forming device 105 is arranged so as to perform vacuum suction until the pulp for forming the second pulp layer 7 formed by crushing a pulp raw fabric 108 is stacked. Note that the airflow forming device 105 may be arranged so as to perform vacuum suction until the second liquid-diffusible fiber sheet 8 is supplied.

An SAP feeder 106 supplies an SAP as the SAP of the first absorbent body 4. Since the first liquid-diffusible fiber sheet 2 is sucked onto the mesh-shaped conveyance belt 104, there is no possibility that the crushed SAP is sucked by the airflow forming device 105.

A thermally-fusible fiber supply device 107 supplies the thermally-fusible fiber 5 toward the mesh-shaped conveyance belt 104. Thus, the thermally-fusible fiber 5 is supplied onto the first absorbent body 4. Subsequently, the SAP feeder 106 in the subsequent stage crushes the SAP and supplies the crushed SAP as the SAP of the second absorbent body 6 onto the thermally-fusible fiber 5. The SAP of the second absorbent body 6 is provided at the skin surface side, whereby the SAP is crushed by the SAP feeder 106 in the subsequent stage in order to make the SAP smaller. Accordingly, it is possible to reduce a user's unpleasant feeling caused by the SAP of the second absorbent body 6 when the user wears the absorbent article 1.

Note that since the average particle size of the SAP of the second absorbent body 6 is larger than the average particle size of the SAP of the first absorbent body 4, the SAP of the second absorbent body 6 may be supplied without crushing the SAP. Further, the average particle size of the SAP of the first absorbent body 4 and the average particle size of the SAP of the second absorbent body 6 may be identical.

The second liquid-diffusible fiber sheet 8 unwound from a liquid-diffusible fiber sheet raw fabric 109 is conveyed on the mesh-shaped conveyance belt 104 along the conveying direction, and is pressed by a pressing roller 110 to form a configuration of the multi-ply sheet 9 (not illustrated in Fig. 2). Although the number of the pressing rollers 110 is one in Fig. 2, another pressing roller may be provided below the conveyance belt 104.

The surface of the multi-ply sheet 9 is formed into an uneven shape by a pair of embossing rollers 111. The surface area of the multi-ply sheet 9 is increased due to the uneven shape, so that it is possible to increase the area for absorbing body fluids. Note that since it is sufficient as long as recesses and protrusions are formed on the surface of the second liquid-diffusible fiber sheet 8, the upper roller of the pair of embossing rollers 111 may be an embossing roller and the lower roller may be a flat roller. Note that, as the embossing shape, various shapes such as vertical, horizontal, wavy, cross, and lattice shapes can be used.

The multi-ply sheet 9 that has passed through the pair of embossing rollers 111 passes through the heating device 112. The heating device 112 heats the multi-ply sheet 9 so as to melt the low melting point component of the thermally-fusible fiber 5. The heat-resistant temperature of the SAP of the first absorbent body 4 and the heat-resistant temperature of the SAP of the second absorbent body 6 are higher than the temperature at which the low melting point component of the thermally-fusible fiber 5 is melted. Therefore, the SAP performance of the first absorbent body 4 and the SAP performance of the second absorbent body 6 are not deteriorated by heating by the heating device 112 and the heating device 114 described below. The multi-ply sheet 9 that has passed through the heating device 112 passes through a pair of embossing rollers 113. An uneven shape is again formed on the surface of the multi-ply sheet 9 by the pair of embossing rollers 113, which makes the multi-ply sheet 9 flexible. Further, the pair of embossing rollers 113 presses the melted thermally-fusible fiber 5 against the SAP of the first absorbent body 4 and the SAP of the second absorbent body 6, so that it is possible to reliably ensure the fixing of the SAP by the thermally-fusible fiber 5. Particularly, as a result of pressing of the pair of embossing rollers 113, the SAP of the second absorbent body 6 above the thermally-fusible fiber 5 can be fixed by the thermally-fusible fiber 5.

When it is not necessary to form the uneven shape on the surface of the multi-ply sheet 9 again, the pair of embossing rollers 113 may be a pair of flat rollers.

The multi-ply sheet 9 that has passed through the pair of embossing rollers 113 passes through a pair of embossing rollers 115 after being heated by the heating device 114. The multi-ply sheet 9 is thus heated and pressed, so that the multi-ply sheet 9 can be made flexible and thin. Further, pressing of the pair of embossing rollers 115 makes it possible to fix the SAP more reliably by the thermally-fusible fiber 5. Note that the pair of embossing rollers 115 may be a pair of flat rollers. In the heating device 112 and the heating device 114, the heat treatment with hot air is usually performed, and the pressing treatment may be performed before and/or after the heat treatment.

As described above, according to the present embodiment, since the first absorbent body 4 and the second absorbent body 6 are provided between the first pulp layer 3 and the second pulp layer 7, it is possible to reduce the risk that the user has a graininess feeling (grainy feeling) of SAP. Further, the SAP of the second absorbent body 6 is crushed, so that it is possible to reduce the risk that the user has a graininess feeling (grainy feeling) of SAP.

Furthermore, the first absorbent body 4 and the second absorbent body 6 are fixed by the thermally-fusible fiber 5, so that it is possible to reduce the risk that the user has an unpleasant feeling due to the transfer of the first absorbent body 4 and the second absorbent body 6.

In addition, since the thermally-fusible fiber 5 is present between the first absorbent body 4 and the second absorbent body 6, larger amounts of the first absorbent body 4 and the second absorbent body 6 can be retained by the thermally-fusible fiber 5. Thus, the amount of body fluids to be absorbed by the absorbent article 1 can be increased, and the absorption rate of body fluids can also be improved. The crushed pulp constituting the first pulp layer 3 and the second pulp layer 7 is usually fixed with a binder such as CMC or PVA.

An absorbent article is produced by the above method (so-called air-laid method using an air-laid forming apparatus), so that it is possible to join and integrate the first liquid-diffusible fiber sheet 2, the first pulp layer 3, the first absorbent body 4, the second absorbent body 6, the second pulp layer 7, and the second liquid-diffusible fiber sheet 8 without using a hot-melt adhesive or the like by layering respective layers thereof, and passing the layers through the heating devices 112 and 114 to heat the layers. The porous portion of the SAP is less covered with the thermally-fusible fiber 5 compared to the hot melt, and thus there is less risk of degrading the absorption performance of SAP. Then, the absorbent article has a thin sheet thickness and excellent absorbency of body fluids, as compared to the absorbent article obtained by joining and integrating respective layers by the hot melt. In the above embodiment, the case where the SAP and the thermally-fusible fiber are supplied separately is shown, but the SAP and the thermally-fusible fiber may be mixed and supplied.

### Second Embodiment

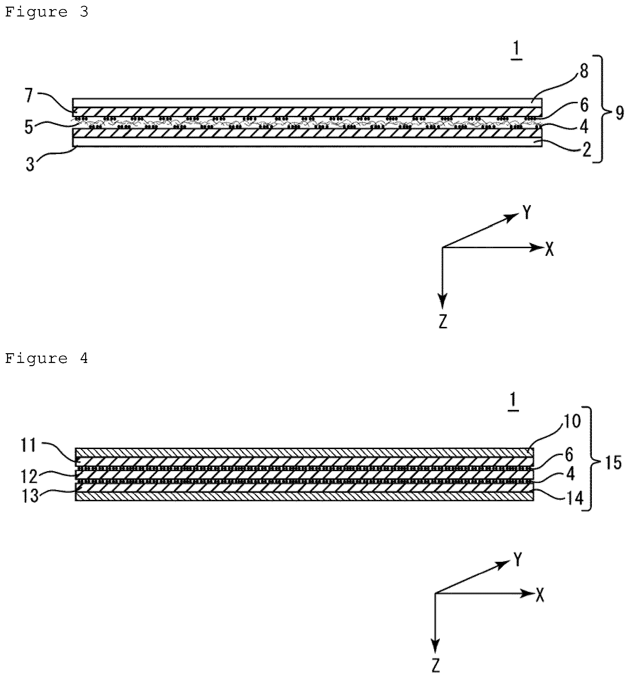

A second embodiment is described below, and the same configurations as the configurations in the first embodiment are referred to by the same reference signs, and the descriptions thereof are omitted. Fig. 3 is a cross-sectional view of the absorbent article 1 of the second embodiment, and the description is continued hereinbelow with reference to Fig. 3.

In the second embodiment, the arrangement of the SAP of the first absorbent body 4 and the SAP of the second absorbent body 6 is different from the arrangement of the SAP of the first absorbent body 4 and the SAP of the second absorbent body 6 in the first embodiment. As is clear from Fig. 3, the first absorbent body 4 and the second absorbent body 6 have an SAP provided region and an SAP non-provided region. As described above, the SAP non-provided region is present, so the absorbent article 1 of the second embodiment can prevent inhibition of body fluid absorption due to gel blocking that occurs when the SAP absorbs body fluids. Note that the SAP provided region and the SAP non-provided region are not limited to the example illustrated in Fig. 3, and can be set as appropriate.

Further, when viewed along the Z-direction in Fig. 3, the SAP provided region in the second absorbent body 6 is present corresponding to the SAP non-provided region in the first absorbent body 4. Thus, the SAP provided region in the second absorbent body 6 can absorb the body fluids that have passed through the SAP non-provided region in the first absorbent body without being affected by gel blocking.

### Third Embodiment

A third embodiment is described below, and the same configurations as the configurations in the first and second embodiments are referred to by the same reference signs, and the descriptions thereof are omitted or simplified. Fig. 4 is a cross-sectional view of the absorbent article 1 of the third embodiment, and the description is continued hereinbelow with reference to Fig. 4.

The absorbent article 1 is a multi-ply sheet 15 in which a first nonwoven fabric 10, a first mixed fiber 11 mixed with a plurality of fibers, the second absorbent body 6, a second mixed fiber 12 mixed with a plurality of fibers, the first absorbent body 4, a third mixed fiber 13 mixed with a plurality of fibers, and a second nonwoven fabric 14 are layered in this order from the skin surface side of the wearer.

### (Nonwoven Fabric)

Since the first nonwoven fabric 10 is arranged at the skin surface side, a hydrophilic nonwoven fabric is used. As the nonwoven fabric, various nonwoven fabrics such as spunbonded nonwoven fabrics, spunlaced nonwoven fabrics, air-through nonwoven fabrics, chemical nonwoven fabrics, and melt blown nonwoven fabrics can be used. In present embodiment, the spunbonded nonwoven fabrics are used. The first nonwoven fabric 10 has a basis weight of 8 g/m² to 15 g/m².

Since the second nonwoven fabric 14 is arranged at the non-skin surface side, a water repellent nonwoven fabric is used. As the nonwoven fabric, various nonwoven fabrics such as spunbonded nonwoven fabrics, spunlaced nonwoven fabrics, air-through nonwoven fabrics, chemical nonwoven fabrics, and melt blown nonwoven fabrics can be used. In present embodiment, the spunbonded nonwoven fabrics are used. The second nonwoven fabric 14 has a basis weight of 8 g/m² to 15 g/m². In the present embodiment, the first nonwoven fabric 10 is the cover sheet at the side of the second absorbent body 6, and the second nonwoven fabric 14 is the cover sheet at the side of the first absorbent body 4.

### (Mixed Fiber)

The first mixed fiber 11, the second mixed fiber 12, and the third mixed fiber 13 are a mixture of the crushed pulp and the thermally-fusible fiber 5 in the present embodiment. The first mixed fiber 11 has a basis weight of 8 g/m² to 15 g/m², and contains a mixture of the crushed pulp in a proportion of 5% to 15% and the thermally-fusible fiber 5 in a proportion of 85% to 95%. Since the first mixed fiber 11 is arranged at the skin surface side, it is necessary to quickly transfer body fluids to the second absorbent body 6. The crushed pulp retains the body fluids, whereas the thermally-fusible fiber 5 does not retain the body fluids. Thus, in the first mixed fiber 11, the mixing proportion of the crushed pulp is reduced, and the basis weight of the crushed pulp is smaller than the basis weights of the second mixed fiber 12 and the third mixed fiber 13. Note that the first mixed fiber 11 may be a single fiber layer containing the crushed pulp in a proportion of 0% and the thermally-fusible fiber 5 in a proportion of 100%.

The second mixed fiber 12 has a basis weight of 35 g/m² to 65 g/m², preferably 45 g/m² to 55 g/m², and contains a mixture of the crushed pulp in a proportion of 65% to 85% and the thermally-fusible fiber 5 in a proportion of 15% to 35%. The second mixed fiber 12 transfers the body fluids that have not been absorbed by the second absorbent body 6 to the first absorbent body 4, and retains the body fluids that have not been absorbed by the second absorbent body 6 by increasing the amount of the crushed pulp to be mixed, whereby the time for transferring the body fluids to the first absorbent body 4 is controlled. As a result, the first absorbent body 4 suppresses the inhibition of body fluid absorption due to gel blocking.

The third mixed fiber 13 has a basis weight of 35 g/m² to 65 g/m², preferably 45 g/m² to 55 g/m², and contains a mixture of the crushed pulp in a proportion of 65% to 85% and the thermally-fusible fiber 5 in a proportion of 15% to 35%. Since the third mixed fiber 13 retains the body fluids that have not been absorbed by the first absorbent body 4, the amount of the crushed pulp to be mixed is increased. Thus, the amount of the crushed pulp of the third mixed fiber 13 may be larger than the amount of the crushed pulp of the second mixed fiber 12.

Note that the second liquid-diffusible fiber sheet 8 may be used instead of the first nonwoven fabric 10, and the first liquid-diffusible fiber sheet 2 may be used instead of the second nonwoven fabric 14.

Note that the basis weight of the SAP of the first absorbent body 4 and the basis weight of the SAP of the second absorbent body 6 are in a range of 100 g/m² to 200 g/m² respectively, and preferably approximately 150 g/m². As described above, the basis weight of the SAP is larger than the total of the basis weights of the first mixed fiber 11, the second mixed fiber 12, and the third mixed fiber 13, and further is larger than the total of the basis weights of the first mixed fiber 11, the second mixed fiber 12, the third mixed fiber 13, the first nonwoven fabric 10, and the second nonwoven fabric 14. For example, assuming that the total of the basis weights of the SAP of the first absorbent body 4 and the SAP of the second absorbent body 6 is 300 g/m², and the total of the basis weights of the first mixed fiber 11, the second mixed fiber 12, and the third mixed fiber 13 is 110 g/m², the total of the basis weights of the SAP of the first absorbent body 4 and the SAP of the second absorbent body 6 is equal to or more than 2 times, equal to or more than 2.5 times, and less than 3 times the total of the basis weights of the first mixed fiber 11, the second mixed fiber 12, and the third mixed fiber 13.

The thermally-fusible fiber 5 in the first mixed fiber 11, the second mixed fiber 12, and the third mixed fiber 13 have a function of fixing the crushed pulp and the SAP. The thermally-fusible fiber 5 is preferably a composite fiber having a core-sheath structure, and preferably a composite fiber in which the sheath portion of the core-sheath structure is made of a resin having a lower melting point than the core portion.

Note that the first mixed fiber 11, the second absorbent body 6, the second mixed fiber 12, the first absorbent body 4, and the third mixed fiber 13 are present as layers separated from one another, but are not limited to this case, and some adjacent layer portions may be overlapped.

### (Multi-ply Sheet Production Method)

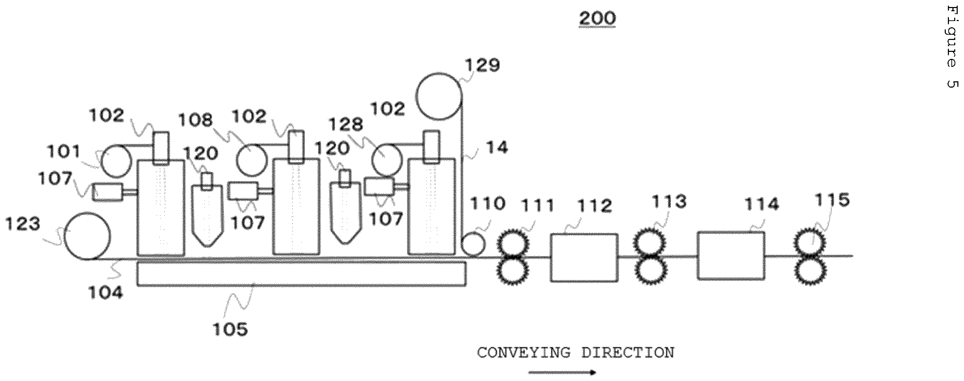

The method for producing the absorbent article 1 of the third embodiment is described below, and the same configurations as the configurations of the production line 100 of Fig. 2 are referred to by the same reference signs, and the descriptions thereof are omitted or simplified. Fig. 5 is a diagram illustrating a production line 200 for the multi-ply sheet 15.

In the third embodiment, the thermally-fusible fiber supply device 107 is connected to the defibration device 102 in order to stack the first mixed fiber 11, the second mixed fiber 12, and the third mixed fiber 13. However, any method may be used as long as the crushed pulp and the thermally-fusible fiber 5 can be mixed. Note that the defibration device 102 and the thermally-fusible fiber supply device 107 may be collectively referred to as "fiber stacking unit".

The first nonwoven fabric 10 (not illustrated in Fig. 5) unwound from a first nonwoven raw fabric 123 is conveyed on the mesh-shaped conveyance belt 104 along the conveying direction. In a state where the first nonwoven fabric 10 having air permeability is sucked by the airflow forming device 105, the crushed pulp and the thermally-fusible fiber 5 are sucked by the airflow forming device 105 through the first nonwoven fabric 10.

An SAP supply device 120 supplies the SAP of the second absorbent body 6.

Due to the suction of the airflow forming device 105, a part of the SAP of the second absorbent body 6 enters the crushed pulp and the thermally-fusible fiber 5, and the rest of the SAP of the second absorbent body 6 is layered on the crushed pulp and the thermally-fusible fiber 5. The SAP that has entered the crushed pulp and the thermally-fusible fiber 5 gives a rigid feeling after absorbing the body fluids, whereas the SAP layered on the crushed pulp and the thermally-fusible fiber 5 gives a soft feeling after absorbing the body fluids. Consequently, the absorbent article 1 that provides comfortable wearing (fitting) can be realized by appropriately controlling the amount of the SAP entering the crushed pulp and the thermally-fusible fiber 5 and the amount of the SAP layered on the crushed pulp and the thermally-fusible fiber 5.

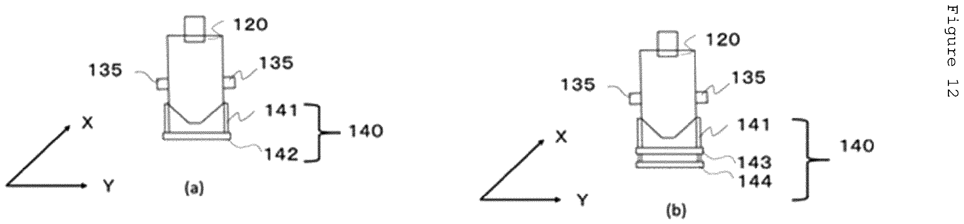

Note that a mesh is formed on the bottom surface of each of the two SAP supply devices 120 according to the size of the SAP to be supplied, and the SAP is supplied from the mesh. Further, the SAP can be supplied more evenly by providing the SAP supply device 120 with an oscillator and oscillating the SAP supply device 120. In this case, a metal member (for example, a spherical shape) is placed in the SAP supply device 120, and the metal member also oscillates, so that the SAP can be supplied evenly. Note that the oscillator of the SAP supply device 120 is illustrated in Fig. 12 to be described below.

Subsequently, the crushed pulp and the thermally-fusible fiber 5 are stacked on the SAP being the second absorbent body 6 from the fiber stacking unit that defibrates the pulp raw fabric 108, and the SAP to be the first absorbent body 4 is supplied to the crushed pulp and the thermally-fusible fiber 5. Due to the suction of the airflow forming device 105, a part of the SAP of the first absorbent body 4 enters the crushed pulp and the thermally-fusible fiber 5, and the rest of the SAP of the first absorbent body 4 is layered on the crushed pulp and the thermally-fusible fiber 5. Therefore, as described above, it is possible to realize the absorbent article 1 that is comfortable to wear.

The crushed pulp and the thermally-fusible fiber are stacked on the SAP being the first absorbent body 4 from the fiber stacking unit that defibrates a pulp raw fabric 128. After that, the second nonwoven fabric 14 from a second nonwoven raw fabric 129 is supplied. The heating device 112 supplies, for example, hot air (for example, at a temperature of 110°C to 150°C) from the second nonwoven fabric 14 side toward the first nonwoven fabric 10 so as to melt the low melting point component of the thermally-fusible fiber, as described above. In this case, hot air is supplied to the thermally-fusible fiber 5 in the second mixed fiber 12 and the first mixed fiber 11 in a state where heat is taken away by the SAP of the first absorbent body 4 and the SAP of the second absorbent body 6. Thus, the high melting point component of the thermally-fusible fiber 5 does not melt, and the multi-ply sheet 15 does not become plastic and rigid due to the melting of the thermally-fusible fiber 5, so that it is possible to realize the absorbent article 1 having a good texture. Further, as a result of melting of the low melting point component of the thermally-fusible fiber, the thermally-fusible fiber rises and forms a net shape. The net-shaped thermally-fusible fiber can retain the SAP. When the absorbent article 1 is produced using the production line 200 of Fig. 5, the SAP of the first absorbent body 4 at the non-skin surface side is expanded greatly when the body fluids are absorbed, whereas the SAP of the second absorbent body 6 at the skin surface side is less expanded even when the body fluids are absorbed, and remains flat. Consequently, the skin surface side has no recesses and protrusions and is flat, it is possible to realize the absorbent article 1 that provides a pleasant feeling against the skin.

Note that, in the production line 200 of Fig. 5, the SAP supply device 120 is provided between fiber stacking units, but the SAP supply device 120 may be incorporated in each of the fiber stacking units. Further, the SAP supply device 120 may be provided between the fiber stacking units, and may be combined with a fiber stacking unit in which the SAP supply device 120 is incorporated.

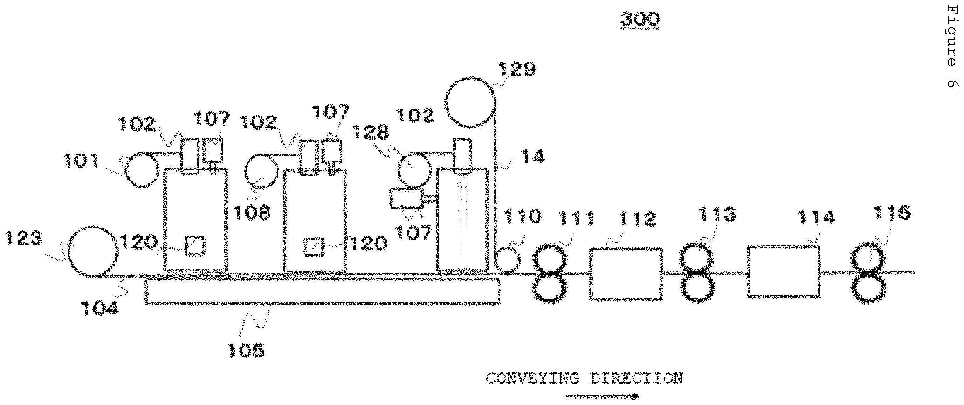

Fig. 6 is a diagram illustrating a production line 300 in which the SAP supply device 120 is incorporated in each fiber stacking unit.

As illustrated in Fig. 6, two fiber stacking units with the SAP supply device 120 incorporated are arranged at an upstream side in the conveying direction, and one fiber stacking unit without the SAP supply device 120 is arranged at a downstream with respect to the two fiber stacking units. In the fiber stacking units with the SAP supply device 120 incorporated, the crushed pulp, the thermally-fusible fiber 5, and the SAP are mixed, and as described above, the absorbent article 1 gives a rigid feeling after absorbing the body fluids. Therefore, the fiber stacking unit with the SAP supply device 120 incorporated is mixed into the fiber stacking unit without the SAP supply device 120, so that it is possible to realize the absorbent article 1 that is comfortable to wear.

Note that the attaching position of the SAP supply device 120 in the fiber stacking unit is adjusted, so that it is possible to control the feel of the absorbent article 1 after absorbing the body fluids.

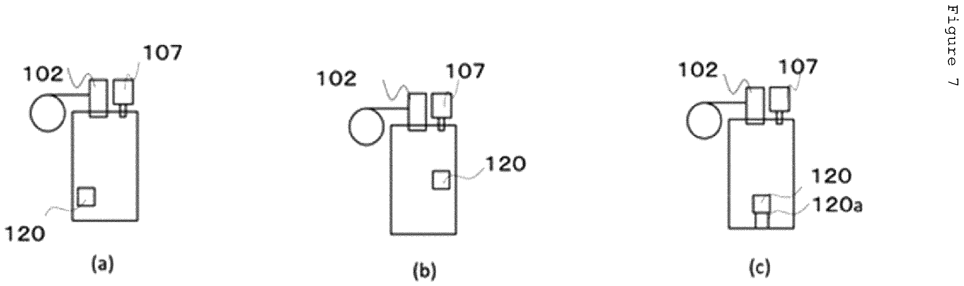

Fig. 7 shows diagrams illustrating arrangement examples of the SAP supply device 120. When the SAP supply device 120 is placed at the left side (upstream side in the conveying direction) as illustrated in Fig. 7 (a), the SAP is supplied to the first nonwoven fabric 10 before the crushed pulp and the thermally-fusible fiber 5 are supplied to the first nonwoven fabric 10. Thus, the absorbent article 1 becomes rigid. On the contrary, when the SAP supply device 120 is arranged at the right side (downstream side in the conveying direction), the absorbent article 1 becomes soft. Note that it is also possible to control the rigidity and softness (gel feeling) of the absorbent article 1 by changing the height at which the SAP supply device 120 is mounted. As illustrated in Fig. 7(b), the SAP supply device 120 is provided slightly above the SAP supply device 120 at the left side, the SAP from the SAP supply device 120 is supplied to not only the left side but also the center due to an influence of the airflow by the airflow forming device 105. Note that, as illustrated in Fig. 7(c), a protective plate 120a may be provided so as not to be influenced by the airflow by the airflow forming device 105.

In the first to third embodiments mentioned above, the pulp raw fabric and the nonwoven raw fabric are used as raw fabric rolls. The pulp raw fabric and the nonwoven raw fabric each can be wound on a roll; up to 7,000 to 9000 m per roll. Meanwhile, an air-laid raw fabric or an air-through nonwoven raw fabric is wound on a roll; up to 2000 m per roll. The roll exchange times are reduced by using the pulp raw fabric and the nonwoven raw fabric, so the production amount can be increased. Further, the frequency of taping rolls together is low, and it is possible to reduce the time and effort required to detect an absorbent article having a joint between rolls and eliminate the absorbent article as a defective product. Furthermore, a solvent-based substance such as a hot melt is not used to joint respective layers, so that the safety of the absorbent article 1 can be improved. In addition, it is possible to suppress the generation of odor caused by the solvent-based substance such as a hot melt. Further, when the SAP is fixed by the hot melt, the hot melt is often spray-coated, the porous portion of the SAP is covered with the hot melt, which may reduce the absorption performance of the SAP. An absorbent article is produced by the above method, so that it is possible to join and integrate respective layers without using a hot-melt adhesive or the like. When the SAP is retained by the thermally-fusible fiber 5, the porous portion of the SAP is less covered with the thermally-fusible fiber 5 compared to the hot melt, and thus there is less risk of degrading the absorption performance of SAP. Then, the absorbent article has a thin sheet thickness and excellent absorbency of body fluids, as compared to the absorbent article obtained by joining and integrating respective layers by the hot melt.

### Fourth Embodiment

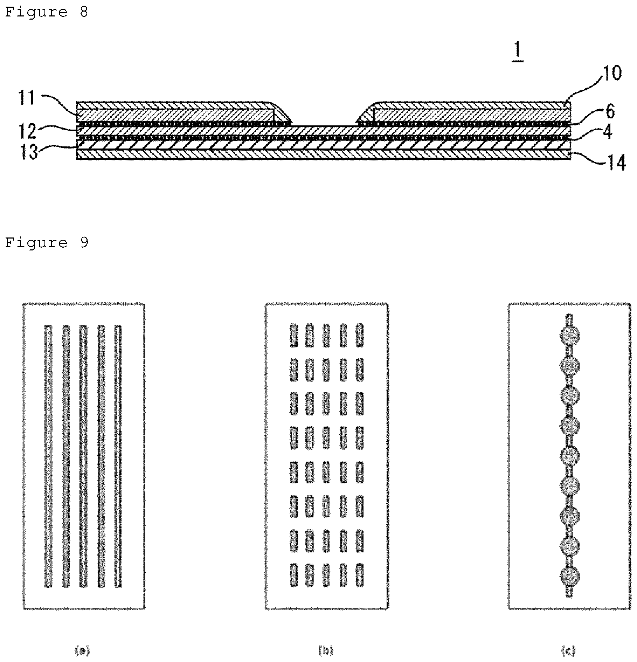

A fourth embodiment is described below, and the same configurations as the configurations in the first to third embodiments are referred to by the same reference signs, and the descriptions thereof are omitted or simplified. Fig. 8 is a cross-sectional view of the absorbent article 1 of the fourth embodiment, and the description is continued hereinbelow with reference to Fig. 8.

In the present embodiment, a region without the first absorbent body 4 and a region without the second absorbent body 6 are linearly provided. The region without the first absorbent body 4 and the region without the second absorbent body 6 may be formed as groove portions or notch (slit) portions. The region without the first absorbent body 4 and the region without the second absorbent body 6 can be formed by masking a part of the mesh on the bottom surface of the SAP supply device 120 of the production line 200 illustrated in Fig. 5. Alternatively, a spherical or triangular member may be arranged below the SAP supply device 120 as a masking member to form the region without the first absorbent body 4 and the region without the second absorbent body 6. In this case, it may be configured that, in addition to the first absorbent body 4 and the second absorbent body 6, at least one of the first mixed fiber 11, the second mixed fiber 12, and the third mixed fiber 13 is not stacked. The size of the groove portion can be varied depending on the position where the masking member is arranged.

Alternatively, the region without the first absorbent body 4 and the region without the second absorbent body 6 may be provided by layering the third mixed fiber 13, the first absorbent body 4, the second mixed fiber 12, the second absorbent body 6, and the first mixed fiber 11 on the second nonwoven fabric 14, and then removing the first mixed fiber 11 from the third mixed fiber 13, or removing the first mixed fiber 11 from the first absorbent body 4. As this removal method, a high pressure air blowing method, a vacuum absorption method or the like can be used. Instead of such a non-contact removal method, a contact method may be used, in which the mechanical parts are transferred from above to below in order to remove, for example, the first mixed fiber 11 from the third mixed fiber 13. In both the contact method and the non-contact method, ON-OFF control enables more precise control than the above-mentioned masking, and it is possible to intermittently form the region without the first absorbent body 4 and the region without the second absorbent body 6. Further, a high pressure air supply head, a vacuum suction head, and the contact portion of the mechanical parts are moved in the left-right direction, so that the region without the first absorbent body 4 and the region without the second absorbent body 6 can be formed into a curved shape, or an arbitrary shape.

The number of the regions without the first absorbent body 4 and the number of the regions without the second absorbent body 6 to be provided are not limited to one, and may be plural. In the case of providing a plurality of regions, the regions may have identical or different shapes. Further, as illustrated in Figs. 9 (a) to 9 (c), each region may have an intermittent shape, or may have a shape having a different width regularly or irregularly.

As described above, in the region without the first absorbent body 4 and the region without the second absorbent body 6, the inhibition of body fluid absorption due to gel blocking, which occurs when SAP absorbs the body fluids, does not occur. Accordingly, it is possible to produce an absorbent body with a large amount of SAP, and it is possible to realize the absorbent article 1 having a large absorption capacity and a high absorption rate. Note that each groove portion (recess portion) is emphasized in Fig. 8, and an actual size of the recess portion is difficult to visibly recognize.

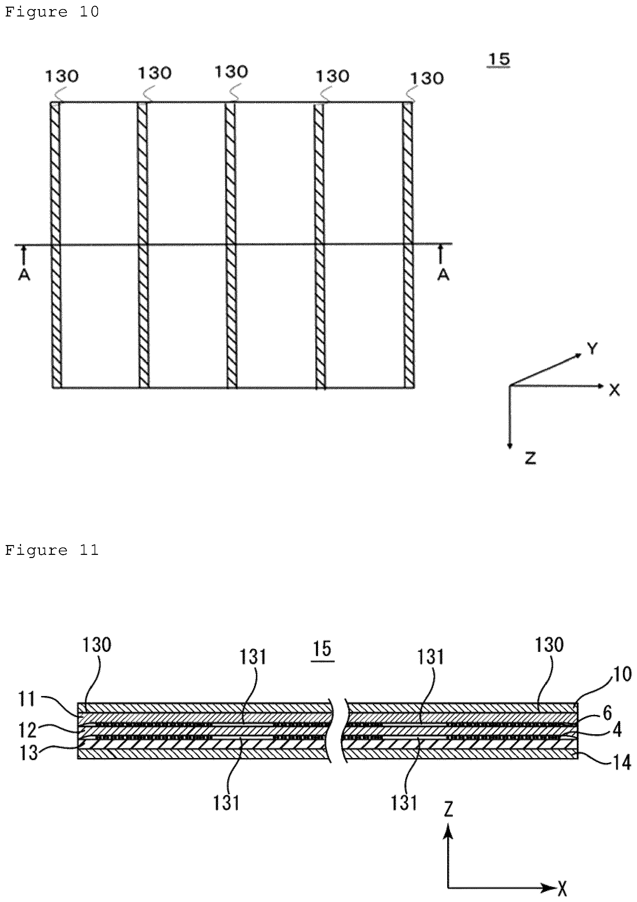

Fig. 10 is a multi-ply sheet 15 produced on the production line 200 of Fig. 5, and Fig. 11 is a diagram illustrating an A-A cross section of the multi-ply sheet 15 of Fig. 10. As illustrated in Figs. 10 and 11, joint portions 130 are formed in the multi-ply sheet 15. Note that hatching is provided in the joint portions 130 in Fig. 10 in order to make the joint portions 130 easy to understand. The joint portions 130 are regions where the first absorbent body 4 and the second absorbent body 6 are absent, particularly regions where the SAP is substantially absent. The first nonwoven fabric 10, the first mixed fiber 11, and the second mixed fiber 12, the third mixed fiber 13, and the second nonwoven fabric 14 are arranged in this order from the water absorption side. The low melting point component of the thermally-fusible fiber is melted by supplying hot air in a range of 110°C to 130°C by the heating device 112 of Fig. 5, whereby the first nonwoven fabric 10, the first mixed fiber 11, and the second mixed fiber 12, the third mixed fiber 13, and the second nonwoven fabric 14 are joined. Note that the joint portions 130 may be formed by heat-bonding the first nonwoven fabric 10 and the second nonwoven fabric 14 (omitting the first mixed fiber 11, the second mixed fiber 12, and the third mixed fiber 13). Further, a pressing treatment using pressing rollers or the like may be performed before and/or after the heating with hot air. The pressing treatment may be applied to the entire multi-ply sheet, and it is particularly preferable to apply the pressing treatment to substantially the SAP-absent region.

Then, the joint portions 130 (excluding the joint portions 130 at both ends) are slit by a slitter to obtain the absorbent article 1. The joint portions 130 are thus formed by thermal fusion, so that it is possible to prevent the first absorbent body 4 and the second absorbent body 6 from spilling out in the horizontal direction (X-direction) from the regions where the first absorbent body 4 and the second absorbent body 6 are present. Note that when the first mixed fiber 11, the second mixed fiber 12, and the third mixed fiber 13 are omitted from the joint portions 130, a mesh may be formed in which the masking member mentioned above is formed on the bottom surface of the thermally-fusible fiber supply device 107 of Fig. 5. In the present embodiment, since the joint portions 130 can be formed without using a device such as an ultrasonic device, it is easy to produce the absorbent article 1 and it is possible to produce the absorbent article 1 which is inexpensive. Note that, although the multi-ply sheet 15 is illustrated in a simplified manner in Fig. 10, the dimension in the X-direction can be set to a range of 1800 mm to 2000 mm, and when the dimension of the absorbent article 1 in the X-direction is set to 100 mm, 18 pieces can be taken, and thus the absorbent article 1 can be produced in large quantities. Further, since the dimension of the absorbent article 1 in the X-direction may be adjusted by adjusting the width of the slitter, the absorbent article 1 used for absorbing small to large amounts can be produced in-line. Examples of the absorbent article 1 used for absorbing large amounts include nursing care products, and the absorbent article 1 used for absorbing large amounts can be used for a hospital bed sheet and also a bed mat laid between a sheet and a bed.

Figs. 12 (a) and 12 (b) are diagrams illustrating outlines of the SAP supply device 120, and each illustrate an oscillator 135 and a masking unit 140. Note that the description is continued hereinbelow with reference to Fig. 12 (a).

The oscillator 135 oscillates the SAP supply device 120, and can be used for various oscillation methods such as an electrical oscillation method and an oscillation method using compressed air. In Figs. 12 (a) and 12 (b), the two oscillators 135 are illustrated, and in this case, it is desirable to displace the mounting positions in the X-direction. The amount of SAP to be supplied from the SAP supply device 120 can be adjusted by adjusting the oscillating amount of the oscillator 135. Note that the number of the oscillators 135 may be one or three or more.

The masking unit 140 blocks the supply of SAP to the joint portions 130 and SAP-absent regions 131 illustrated in Fig. 11, and has an attaching portion 141 and a masking member 142. The attaching portion 141 is a member that attaches the masking unit 140 to the SAP supply device 120.

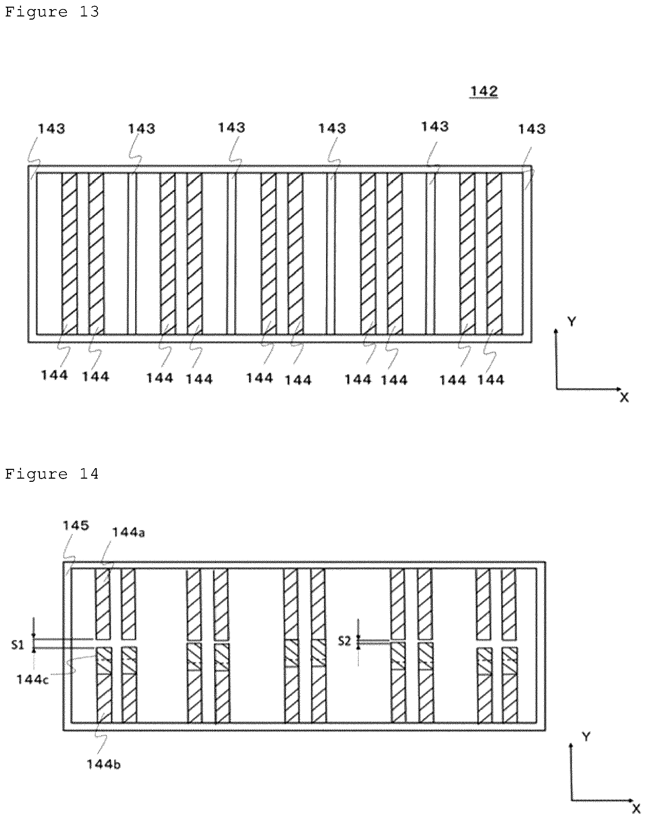

Fig. 13 is a diagram illustrating the masking member 142. The masking member 142 has a first masking portion 143 that blocks the supply of SAP to the joint portions 130, and a second masking portion 144 that blocks the supply of SAP to the SAP-absent regions 131.

The speed of water absorption of the absorbent article 1 can be adjusted by adjusting the width (dimension in the X-direction) and the number of the second masking portions 144. This is because since the SAP is not supplied, the SAP-absent regions 131 do not cause the gel blocking phenomenon due to the swelling of SAP, and urine can be quickly transferred in the Y-direction.

In Fig. 11, the position in the X-direction in which the SAP-absent region 131 is formed in the first absorbent body 4 is identical to the position in the X-direction in which the SAP-absent region 131 is formed in the second absorbent body 6, but it may be configured such that the position in the X-direction in which the SAP-absent region 131 is formed in the first absorbent body 4 is different from the position in the X-direction in which the SAP-absent region 131 is formed in the second absorbent body 6. In this case, it may be configured such that the positions of the two second masking portions 144 in the X-direction are different.

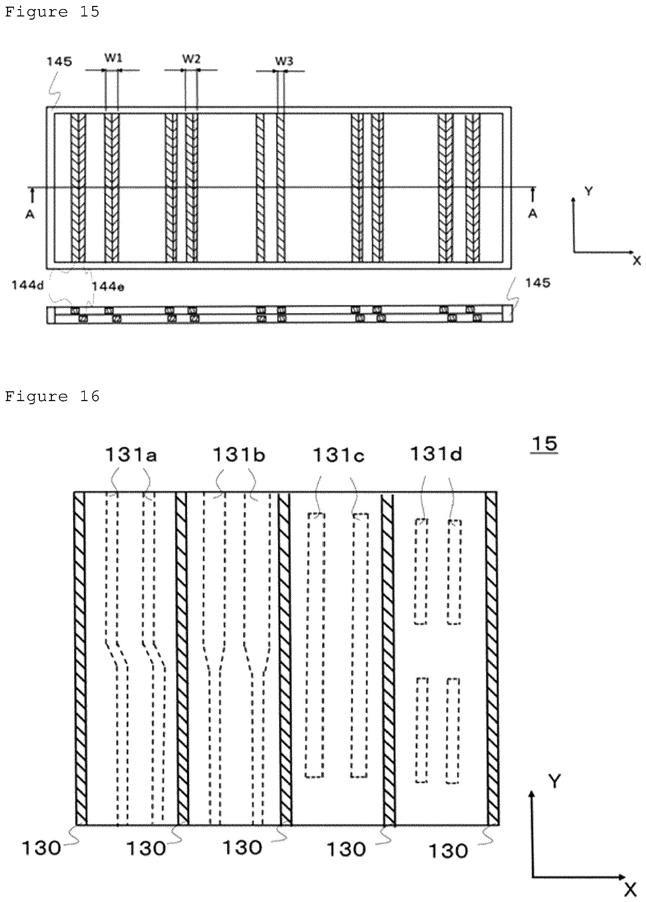

Note that when the second masking portions 144 are movable in the X-direction with respect to the outer frame of the masking member 142, the positions of the SAP-absent regions 131 in the X-direction can be changed. Thus, the SAP-absent regions 131 can be formed so as to be shifted in the X-direction. Fig. 16 is a diagram illustrating examples of the SAP-absent regions 131 with dotted lines. When the second masking portions 144 are movable in the X-direction, SAP-absent regions shifted in the X-direction from the middle as illustrated in SAP-absent regions 131a can be formed.

Note that it may be configured such that the SAP supply device for supplying the SAP to the SAP-absent regions 131 is provided at the upstream side of the defibration device 102 for defibrating the pulp raw fabric 128 in Fig. 5, and the SAP-absent regions 131 are formed intermittently in the Y-direction. When the SAP-absent regions 131 are continuously formed in the Y-direction, there is a risk of urine leakage from the wearer's back when the absorbent article 1 is used as an absorbent article for diapers. Meanwhile, when the SAP-absent regions 131 are intermittently formed in the Y-direction, it is possible to prevent urine leakage from the wearer's back when the absorbent article 1 is used as the absorbent article for diapers.

Further, the first masking portion 143 and the second masking portion 144 may be separated and formed as separate members so that the entire second masking portion 144 is movable in the X-direction.

Fig. 12 (b) illustrates an example in which the first masking portion 143 and the second masking portion 144 are formed as separate members. In Fig. 12 (b), the first masking portion 143 is located above the second masking portion 144, but the first masking portion 143 and the second masking portion 144 may be arranged such that the first masking portion 143 is located below the second masking portion 144.

Fig. 14 is a diagram illustrating an example in which the second masking portion 144 is a separate member from the first masking portion 143. The second masking portion 144 includes a first mask member 144a that is cantilevered by a support member 145, a second mask member 144b that is cantilevered by the support member 145, and a third mask member 144c that can move on the second mask member 144b in the Y-direction.

The third mask member 144c is moved in the Y-direction by a motor, an air cylinder, or the like to form a gap S1 or S2 with the first mask member 144a, or to prevent formation of a gap with the first mask member 144a. When each of the gap S1 and the gap S2 is formed between the third mask member 144c and the first mask member 144a, the SAP from the SAP supply device 120 passes through the gap S1 and the gap S2. SAP-absent regions 131c and SAP-absent regions 131d illustrated by the dotted lines in Fig. 16 are driven so that the third mask member 144c prevents formation of a gap between the third mask member 144c and the first mask member 144a. Note that, in addition to the SAP-absent regions 131c and the SAP-absent regions 131d illustrated by the dotted lines, the above-mentioned gap S1 or S2 is formed and the SAP is supplied. Since the positions of the SAP-absent regions 131c and the SAP-absent regions 131d in the Y-direction can be controlled by driving the third mask member 144c, the SAP-absent regions 131 are not formed at the ends in the Y-direction. Accordingly, when the absorbent article 1 is used as an absorbent article for diapers, urine passing through the SAP-absent regions 131 does not reach the wearer's back.

Fig. 15 is a diagram illustrating an example in which the second masking portion 144 is a separate member from the first masking portion 143, and the A-A cross section is illustrated below. The second masking portion 144 includes a fourth mask member 144d that is supported by the support member 145 so as to be movable in the X-direction, and a fifth mask member 144e that is located below the fourth mask member 144d and is supported by the support member 145 so as to be movable in the X-direction. The fourth mask member 144d and the fifth mask member 144e are moved in the X-direction by a motor, an air cylinder, or the like, whereby the degree of overlap between the fourth mask member 144d and the fifth mask member 144e in the X-direction is adjusted.

When the degree of overlap between the fourth mask member 144d and the fifth mask member 144e in the X-direction is very low (in the case of W1), the widths of the SAP-absent regions 131 in the X-direction become wider. Further, when the degree of overlap between the fourth mask member 144d and the fifth mask member 144e in the X-direction is higher than the above degree (in the case of W2), the widths of the SAP-absent regions 131 in the X-direction become narrower. When the fourth mask member 144d and the fifth mask member 144e are overlapped in the X-direction (in the case of W3), the widths of the SAP-absent regions 131 in the X-direction are further narrowed. In SAP-absent regions 131b illustrated by the dotted line in Fig. 16, the widths of the SAP-absent regions 131 in the X-direction are changed by adjusting the degree of overlap between the fourth mask member 144d and the fifth mask member 144e in the X-direction.

Note that, as can be seen from Fig. 11, the SAP-absent regions 131 can be formed by stacking the first mixed fiber 11, the second mixed fiber 12, and the third mixed fiber 13, and can be formed without a pressing step. Thus, the SAP-absent regions 131 can be easily formed without forming a groove or a notch. Further, in the SAP-absent regions 131, similarly to the joint portions 130, the low melting point component of the thermally-fusible fiber is melted by the heating device 112 of Fig. 5, thereby joining of the first nonwoven fabric 10, the first mixed fiber 11, the second mixed fiber 12, the third mixed fiber 13, and the second nonwoven fabric 14. Accordingly, the SAP between the two SAP-absent regions 131a illustrated in Fig. 16 is prevented from transferring to the SAP-absent regions 131a.

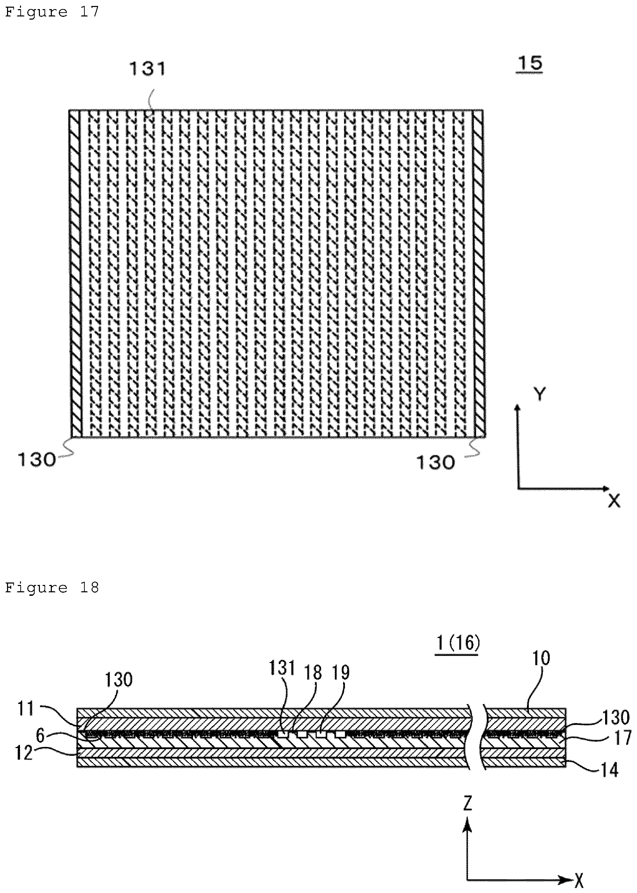

Of the joint portions 130 illustrated in Fig. 11, the joint portions 130 excluding both ends may also be used as the SAP-absent regions 131. Fig. 17 is a diagram illustrating a modified example of the SAP-absent regions 131, and the SAP-absent regions 131 are illustrated by dotted lines. As illustrated in Fig. 17, the SAP-absent regions 131 are formed at a predetermined pitch (for example, at intervals of 10 to 20 mm) along the X-direction, so that the SAP-absent regions 131 can also be used as the joint portions 130. Note that an SAP-absent region is a region in which the SAP is not intentionally supplied and the SAP is not present in practice, but it does not mean that the SAP is not present at all, and a certain amount of SAP may be scattered. An absorbent article is produced by the above method, so that it is possible to join and integrate respective layers without using a hot-melt adhesive or the like, the porous portion of the SAP is less covered with the thermally-fusible fiber 5 compared to the hot melt, and thus there is less risk of degrading the absorption performance of SAP. Therefore, it is possible to obtain an absorbent article having excellent absorbency of body fluids and a thin sheet thickness and excellent absorbency of body fluids, as compared to the absorbent article obtained by joining and integrating respective layers by the hot melt.

### Fifth Embodiment

A fifth embodiment is described below, and the same configurations as the configurations in the first to fourth embodiments are referred to by the same reference signs, and the descriptions thereof are omitted or simplified. Fig. 18 is a cross-sectional view of the absorbent article 1 of the fifth embodiment, and the description is continued hereinbelow with reference to Fig. 18. The absorbent article 1 of the fifth embodiment is a multi-ply sheet 16 in which the first nonwoven fabric 10, the first mixed fiber 11, the second absorbent body 6, an air-through nonwoven fabric 17, the second mixed fiber 12, and the second nonwoven fabric 14 are layered in this order from above. In the present embodiment, the first nonwoven fabric 10 and the second nonwoven fabric 14 are cover sheets for the second absorbent body 6.

The first nonwoven fabric 10 and the second nonwoven fabric 14 are spunbonded nonwoven fabrics each having a basis weight of 8 g/m² to 15 g/m². The first nonwoven fabric 10 is a hydrophilic spunbonded nonwoven fabric, and the second nonwoven fabric 14 may be a hydrophilic spunbonded nonwoven fabric or a water repellent spunbonded nonwoven fabric.

The first mixed fiber 11 and the second mixed fiber 12 each have a basis weight of 8 g/m² to 15 g/m², and contains a mixture of the crushed pulp in a proportion of 0% to 15% and the thermally-fusible fiber 5 in a proportion of 85% to 100%.

In the present embodiment, since the first absorbent body 4 is omitted and only the second absorbent body 6 is used, the basis weight of the SAP of the second absorbent body 6 is in a range of 200 g/m² to 400 g/m², and preferably approximately 300 g/m². Further, in the present embodiment, the SAP-absent region 131 is also formed.

Note that, in the present embodiment, the first absorbent body 4 may be provided between the air-through nonwoven fabric 17 and the second mixed fiber 12.

### (Air-through Nonwoven Fabric)

The air-through nonwoven fabric 17 is a nonwoven fabric formed by blowing hot air on a fiber web containing fusible fibers by a hot air penetration method and fusing the intersections of the thermally-fusible fibers in the web. Since the air-through nonwoven fabric 17 has large gaps as compared to the crushed pulp, the SAP can be retained in the gaps. Further, even when the SAP absorbs the body fluids and swells, the air-through nonwoven fabric 17 enables the SAP to be retained in the gaps. Thus, the absorbent article 1 does not lose its shape due to the transfer of the SAP. In the present embodiment, the basis weight of the air-through nonwoven fabric 17 is in a range of 30 g/m² to 60 g/m², and preferably approximately 45 g/m².

In the air-through nonwoven fabric 17 of the present embodiment, a plurality of protrusion portions 18 and a plurality of recess portions 19 are formed along the conveying direction. The air-through nonwoven fabric 17 is pressed, ultrasonically bonded, or heat-sealed, so that the recess portions 19 can be formed. In the present embodiment, the recess portions 19 along the conveying direction are formed by an embossing roller, and the protrusion portions 18 having a protruding shape relative to the recess portions 19 are formed. As mentioned above, the air-through nonwoven fabric 17 has large gaps, and thus does not have much liquid diffusibility. However, a gap in the Z-direction of the recess portions 19 pressed by the embossing roller is smaller than a gap in the Z-direction of the protrusion portions 18. Thus, the liquid diffusibility of the recess portions 19 is higher than the liquid diffusibility of the protrusion portions 18, whereby the body fluids transfer along the recess portions 19, the body fluids diffuse in the X-direction, and the body fluids tend to transfer in the Y-direction. Therefore, even when the air-through nonwoven fabric 17 is used, it is not necessary to add a sublayer to ensure the liquid diffusibility.

Further, the SAP of the second absorbent body 6 is retained in the protrusion portions 18, but is located on the surfaces of the recess portions 19. When the SAP of the second absorbent body 6 absorbs the body fluids and swells, the SAP retained in the protrusion portions 18 is not able to transfer, and thus becomes rigid as compared to the SAP that is located on the surfaces of the recess portions 19 and movable. Accordingly, the rigidity and softness of the absorbent article 1 can be adjusted by adjusting the sizes of the protrusion portions 18 and the recess portions 19. In the absorbent article 1 for women and babies, the softness is required when the SAP swells, so it is desirable to increase the widths of the recess portions 19 in the X-direction or increase the depths of the recess portions 19 in the Z-direction.

The recess portions 19 may be formed continuously along the conveying direction, or may be formed intermittently to prevent urine leakage from the wearer's back.

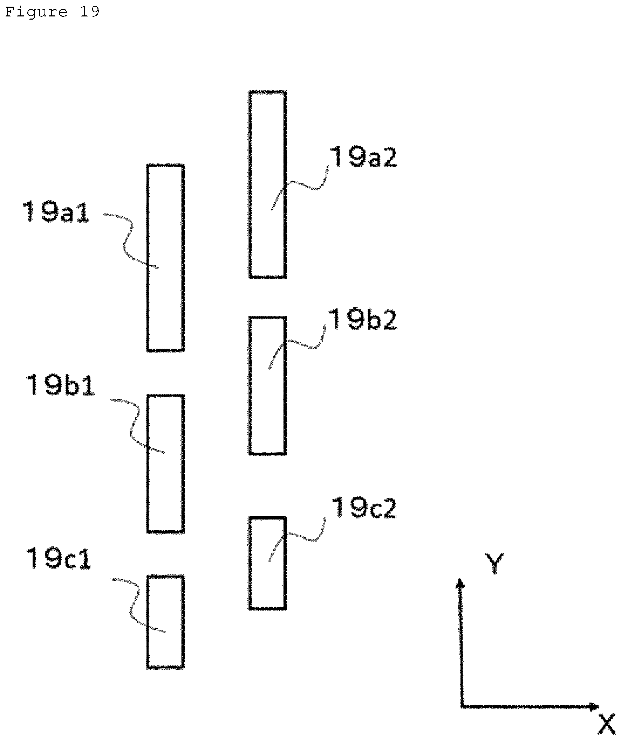

Fig. 19 is a diagram illustrating the details of the recess portions 19, in which recess portions 19a1, 19b1, and 19c1 are formed intermittently in the Y-direction, i.e., the conveying direction. Further, recess portions 19a2, 19b2, and 19c2 are formed so as to be shifted in the X-direction. The length of the recess portion 19a1 (19a2) in the Y-direction is longer than the lengths of the recess portion 19b1 (19b2) and the recess portion 19c1 (19c2) in the Y-direction. The length of the recess portion 19b1 (19b2) in the Y-direction is longer than the length of the recess portion 19c1 (19c2) in the Y-direction. For example, when the lengths of the recess portions 19 in the Y-direction are shortened along the direction of movement of urine, the movement of urine can be slowed down or stopped. Accordingly, it is possible to reduce causing of urine leakage from the wearer's back or abdomen.

Further, in a portion where the recess portions 19 are formed intermittently in the Y-direction, that is, a portion where the recess portion 19 between the recess portion 19a1 and the recess portion 19b1 is not formed, the recess portion 19b2 is formed in the X-direction. Thus, the body fluids diffused in the X-direction in the recess portion 19a1 transfer to the recess portion 19b2 and are absorbed by the second absorbent body 6, which is omitted in Fig. 19, whereby the absorption rate does not decrease. The SAP-absent regions 131 of the present embodiment may have the shapes of the SAP-absent regions 131a to 131d illustrated in Fig. 16, and the number of the SAP-absent regions 131 may be any number.

In the present embodiment as well, similarly to the third embodiment, the basis weight of the SAP of the second absorbent body 6 is larger than the total of the basis weights of the first mixed fiber 11, the second mixed fiber 12, and the air-through nonwoven fabric 17, and further is larger than the total of the basis weights of the first nonwoven fabric 10 and the second nonwoven fabric 14. For example, assuming that the basis weight of the SAP of the second absorbent body 6 is 300 g/m², and the total of the basis weights of the first mixed fiber 11, the second mixed fiber 12, and the air-through nonwoven fabric 17 is 75 g/m², the basis weight of the SAP of the second absorbent body 6 is 4 times the total of the basis weights of the first mixed fiber 11, the second mixed fiber 12, and the air-through nonwoven fabric 17. Assuming that the total of the basis weights of the first nonwoven fabric 10, the first mixed fiber 11, the second mixed fiber 12, the air-through nonwoven fabric 17, and the second nonwoven fabric 14 is 95, the basis weight of the SAP of the second absorbent body 6 is about 3.2 times, i.e., 3 times or more the above total of the basis weights. The absorbent article 1 with a large basis weight of the SAP is obtained.

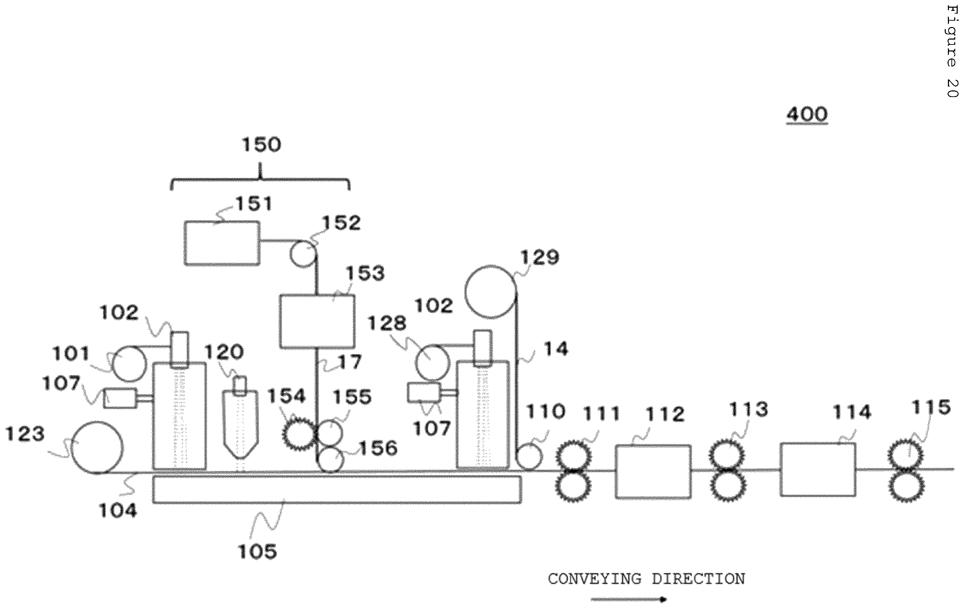

Fig. 20 is a diagram illustrating a production line 400 of the multi-ply sheet 16 according to the fifth embodiment, and the same configurations as the configurations of the production lines 100, 200, and 300 are referred to by the same reference signs, and the descriptions thereof are omitted or simplified.

The first nonwoven fabric 10 (not illustrated in Fig. 20) unwound from the first nonwoven raw fabric 123 is conveyed on the mesh-shaped conveyance belt 104 along the conveying direction. In a state where the first nonwoven fabric 10 having air permeability is sucked by the airflow forming device 105, the crushed pulp and the thermally-fusible fiber are supplied from the fiber stacking unit at the upstream through the first nonwoven fabric 10 in order to form the first mixed fiber 11.

An SAP supply device 120 supplies the SAP of the second absorbent body 6.

In the production line 400, an air-through non-woven fabric production apparatus 150 is incorporated in-line to produce the air-through nonwoven fabric 17.

The air-through non-woven fabric production apparatus 150 includes a carding device 151 and a heating device 153.

Fusible fibers from a raw material supply device (not illustrated) are supplied to the carding device 151. As the fusible fibers, for example, a composite fiber made of polyethylene and polypropylene and a composite fiber made of polyethylene and polyester can be used, but the fusible fibers are not limited to these composite fibers.

The carding device 151 spreads the supplied fibers into an almost uniform state and forms a web which is a fiber aggregate. In the heating device 153, the web conveyed from a conveyance roller 152 is thermally fused by a hot air treatment to form the air-through nonwoven fabric 17.

In the air-through nonwoven fabric 17, the protrusion portions 18 and the recess portions 19 are formed by an embossing roller 154 and a flat roller 155. Then, the air-through nonwoven fabric 17 is conveyed onto the first mixed fiber 11 and pressed by a pressing roller 156. After that, the fiber stacking unit located downstream supply the crushed pulp and the thermally-fusible fiber to form the second mixed fiber 12 on the air-through nonwoven fabric 17. Then, the second nonwoven fabric 14 from the second nonwoven raw fabric 129 is supplied and passed through the treatment of the pair of embossing rollers 111, the pair of embossing rollers 113, the pair of embossing rollers 115, and the heating devices 112 and 114 to produce the multi-ply sheet 16 of the fifth embodiment. Note that the air-through nonwoven fabric 17 shrinks due to the treatment of the heating devices 112 and 114, and thus retains the SAP of the second absorbent body 6 in the protrusion portions 18.

When the air-through nonwoven fabric 17 is used as the raw fabric, the length is about 2000 m per roll as mentioned above, and a raw fabric replacement operation is required. Meanwhile, when the air-through non-woven fabric production apparatus 150 is incorporated in-line in the production line 400, the raw fabric replacement operation can be omitted, and the multi-ply sheet 16 can be continuously produced. Note that, in the production line 400, although the multi-ply sheet 16 is formed by layering from the side of the first nonwoven fabric 10, the multi-ply sheet 16 may be formed by layering from the side of the second nonwoven fabric 14. In the other production lines 100, 200, and 300, it is possible to appropriately change the side from which the multi-ply sheet 16 is layered. Note that, in the production line 400, it is also possible to omit the air-through non-woven fabric production apparatus 150 and use the raw fabric of the air-through nonwoven fabric 17. An absorbent article is produced by the above method, so that it is possible to join and integrate respective layers without using a hot-melt adhesive or the like. The porous portion of the SAP is less covered with the thermally-fusible fiber 5 compared to the hot melt, and thus there is less risk of degrading the absorption performance of SAP. Then, the absorbent article having excellent absorbency of body fluids and a thin sheet thickness and excellent absorbency of body fluids, as compared to the absorbent article obtained by joining and integrating respective layers by the hot melt. In the absorbent article of the present embodiment, the SAP and the crushed pulp are retained in the gaps of the air-through nonwoven fabric, so that it is possible to prevent a decrease in gel feeling when the SAP absorbs water and swells, and a shift between respective layers.

### Sixth Embodiment

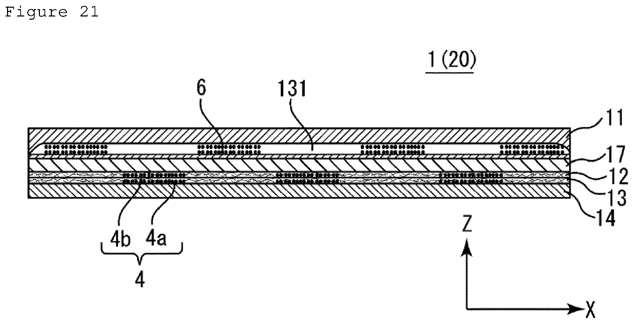

A sixth embodiment is described below, and the same configurations as the configurations in the first to fifth embodiments are referred to by the same reference signs, and the descriptions thereof are omitted or simplified. Fig. 21 is a cross-sectional view of the absorbent article 1 of the sixth embodiment, and the description is continued hereinbelow with reference to Fig. 21. The absorbent article 1 of the sixth embodiment is configured by omitting the first nonwoven fabric 10, and is a multi-ply sheet 20 in which the first mixed fiber 11, the second absorbent body 6, the air-through nonwoven fabric 17, the second mixed fiber 12, the third mixed fiber 13, and the second nonwoven fabric 14 are layered in this order from above. Note that, in the present embodiment, first absorbent body portions 4a and 4b (the first absorbent body 4) are mixed with the second mixed fiber 12 and the third mixed fiber 13.

The first mixed fiber 11 has a basis weight of 8 g/m² to 15 g/m², and contains a mixture of the crushed pulp in a proportion of 0% to 10% and the thermally-fusible fiber in a proportion of 90% to 100%.

The basis weight of the SAP of the second absorbent body 6 is in a range of 100 g/m² to 200 g/m², and preferably in a range of 120 g/m² to 150 g/m². Further, in the present embodiment, the SAP-absent region 131 is also formed.

The basis weight of the air-through nonwoven fabric 17 is in a range of 10 g/m² to 30 g/m², and preferably approximately 20 g/m².

The second mixed fiber 12 has a basis weight of 20 g/m² to 40 g/m², and contains a mixture of the crushed pulp in a proportion of 70% to 90% and the thermally-fusible fiber in a proportion of 10% to 30%.

The third mixed fiber 13 has a basis weight of 10 g/m² to 20 g/m², and contains a mixture of the crushed pulp in a proportion of 30% to 50% and the thermally-fusible fiber in a proportion of 50% to 70%.

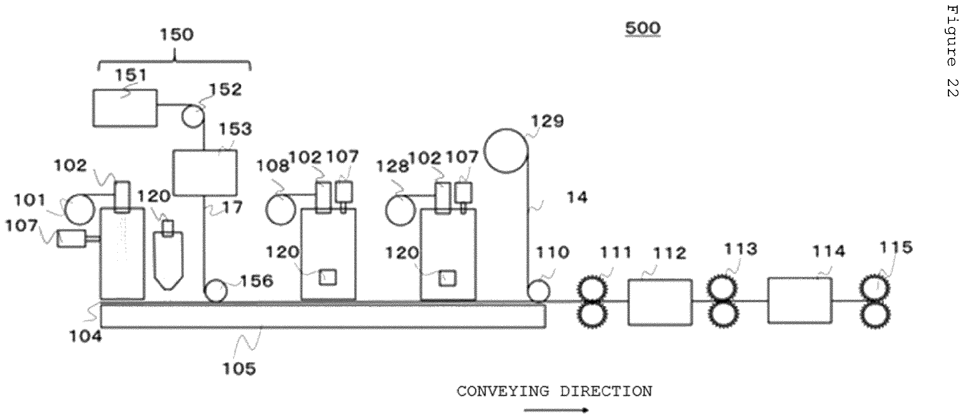

Although the details will be described with reference to Fig. 22, in the present embodiment, two fiber stacking units in which the SAP supply device 120 is incorporated (see Fig. 6) are prepared. One of the two fiber stacking units mixes the second mixed fiber 12 and the SAP constituting the first absorbent body portion 4a and supplies the resultant product, and the other mixes the third mixed fiber 13 and the SAP constituting the first absorbent body portion 4b and supplies the resultant product. Note that the first absorbent body portion 4a and the first absorbent body portion 4b are provided corresponding to the SAP-absent region 131 of the second absorbent body 6, and an SAP-absent region (not illustrated) is formed corresponding to the region where the second absorbent body 6 is present.

Note that the total of the basis weights of the SAP of the first absorbent body portion 4a and the SAP of the first absorbent body portion 4b is in a range of 80 g/m² to 120 g/m², which is smaller than the basis weight of the SAP of the second absorbent body 6.

As described above, in the present embodiment, the SAP region can be formed separately like the second absorbent body 6, and the SAP region can be formed so as to be mixed into the fiber layer, like the first absorbent body portions 4a and 4b. Note that, when the SAP region is formed separately like the second absorbent body 6, it can be said that the SAP region is an independent region even when a certain number of fibers is mixed into the SAP region.

In the second nonwoven fabric 14, a spunbonded nonwoven fabric having a basis weight of 8 g/m² to 12 g/m² is used, and other nonwoven fabrics can also be used.

In the present embodiment, the basis weights of the SAP of the first absorbent body 4 and the SAP of the second absorbent body 6 are approximately 230 g/m², whereas the basis weights of the first mixed fiber 11, the air-through nonwoven fabric 17, and the second mixed fiber 12, the third mixed fiber 13, and the second nonwoven fabric 14 are approximately 90 g/m². The basis weights of the SAP of the first absorbent body 4 and the SAP of the second absorbent body 6 are 2 times or more (2.5 times or more), i.e., less than 3 times the above basis weights.

Fig. 22 is a diagram illustrating a production line 500 of the multi-ply sheet 20, and the same configurations as the configurations of the production lines 100, 200, 300, and 400 are referred to by the same reference signs, and the descriptions thereof are omitted or simplified. Note that, to simplify the drawing, the oscillator 135 and the masking unit 140 provided in the SAP supply device 120 are not illustrated.

The crushed pulp obtained by defibrating the pulp raw fabric 101 by the defibration device 102 and the thermally-fusible fiber from the thermally-fusible fiber supply device 107 are supplied to the conveyance belt 104, and the first mixed fiber 11 is formed.

The SAP from the SAP supply device 120 separated from the fiber stacking unit is supplied to the first mixed fiber 11, and the second absorbent body 6 is formed. Note that, a part of the SAP from the SAP supply device 120 may be mixed with the first mixed fiber 11, but the second absorbent body 6 is formed separately from the first mixed fiber 11 and the air-through nonwoven fabric 17. The layers of SAP are configured separately like the second absorbent body 6, so that it is possible to supply a large amount of SAP and it is possible to make the absorbent article 1 soft to the touch when the SAP absorbs body fluids such as urine.

The air-through nonwoven fabric 17 produced by the air-through non-woven fabric production apparatus 150 is supplied so as to cover the second absorbent body. Note that, although the embossing roller 154 and the flat roller 155 are omitted in Fig. 22, the air-through nonwoven fabric 17 may be embossed or need not be embossed. When recess portions are formed in the air-through nonwoven fabric 17 by embossing or the like, the liquid diffusibility is improved as mentioned above, and the body fluids can be quickly diffused in the plane direction (X-Y plane).

The crushed pulp obtained by defibrating the pulp raw fabric 108 by the defibration device 102 and the thermally-fusible fiber from the thermally-fusible fiber supply device 107, in a state mixed with the SAP from the SAP supply device 120 incorporated to the fiber stacking unit, are supplied onto the air-through nonwoven fabric 17. Then, the second mixed fiber 12 in which the first absorbent body portion 4b is mixed is formed on the air-through nonwoven fabric 17.

Similarly, the crushed pulp obtained by defibrating the pulp raw fabric 128 by the defibration device 102 and the thermally-fusible fiber from the thermally-fusible fiber supply device 107, in a state mixed with the SAP from the SAP supply device 120 incorporated to the fiber stacking unit, are supplied onto the second mixed fiber 12. Then, the third mixed fiber 13 in which the first absorbent body portion 4a is mixed is formed on the second mixed fiber 12. The SAP is thus mixed into the fiber layer, whereby the layer structure of the absorbent article 1 can be reduced and the absorbent article 1 can be made thinner.

The second nonwoven fabric 14 is supplied from the second nonwoven raw fabric 129 onto the third mixed fiber 13, and pressed and heat-treated to produce the multi-ply sheet 20. Note that, although not illustrated in Fig. 21, the joint portions 130 may be provided in the multi-ply sheet 20. In this case, the joint portions 130 can be produced by the heating device 112 and the pair of embossing rollers 113.

Note that, in the production line 500, respective layers are layered sequentially from the first mixed fiber 11 side to form the multi-ply sheet 20, but respective layers may be layered sequentially from the second nonwoven fabric 14 side to form the multi-ply sheet 20. Further, a fiber stacking unit including the defibration device 102 and the thermally-fusible fiber supply device 107 may be provided with a member that masks the supply of the crushed pulp and the thermally-fusible fiber, like the masking unit 140. In this case, the supply of the crushed pulp and the thermally-fusible fiber may be partially blocked or totally blocked. For example, the supply of the crushed pulp and the thermally-fusible fiber may be totally blocked to form the SAP layers separately. An absorbent article is produced by the above method, so that it is possible to join and integrate respective layers without using a hot-melt adhesive or the like, the porous portion of the SAP is less covered with the thermally-fusible fiber 5 compared to the hot melt, and thus there is less risk of degrading the absorption performance of SAP. Therefore, it is possible to obtain an absorbent article having a thin sheet thickness and excellent absorbency of body fluids and the like, as compared to the absorbent article obtained by joining and integrating respective layers by the hot melt.

### Seventh Embodiment

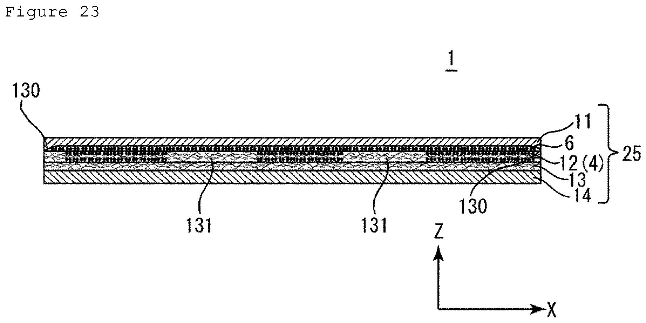

A seventh embodiment is described below, and the same configurations as the configurations in the first to sixth embodiments are referred to by the same reference signs, and the descriptions thereof are omitted or simplified. Fig. 23 is a cross-sectional view of the absorbent article 1 of the seventh embodiment, and the description is continued hereinbelow with reference to Fig. 23. The absorbent article 1 of the seventh embodiment is configured by omitting the first nonwoven fabric 10, and is a multi-ply sheet 25 in which the first mixed fiber 11, the second absorbent body 6, the second mixed fiber 12, the third mixed fiber 13, and the second nonwoven fabric 14 are layered in this order from above. Note that, in the present embodiment, the first absorbent body 4 is mixed into the second mixed fiber 12.

The first mixed fiber 11 has a basis weight of 8 g/m² to 25 g/m², and contains a mixture of the crushed pulp in a proportion of 0% to 20% and the thermally-fusible fiber in a proportion of 80% to 100%.

The basis weight of the SAP of the second absorbent body 6 is in a range of 60 g/m² to 120 g/m², and preferably in a range of 80 g/m² to 100 g/m². Further, in the present embodiment, the SAP-absent regions 131 are not formed, but the SAP-absent regions 131 may be formed.

The second mixed fiber 12 has a basis weight of 30 g/m² to 60 g/m², and contains a mixture of the crushed pulp in a proportion of 70% to 90% and the thermally-fusible fiber in a proportion of 10% to 30%.

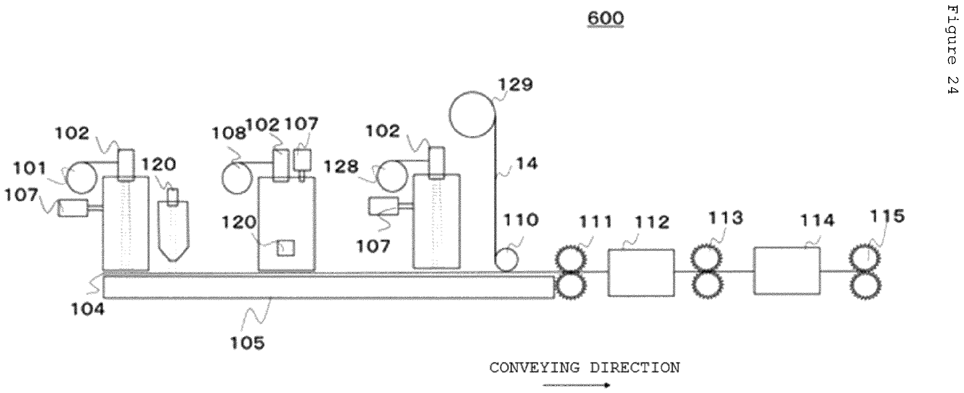

Although the details will be described with reference to Fig. 24, in the present embodiment, one fiber stacking unit with the SAP supply device 120 incorporated (see Fig. 6) is prepared, and the second mixed fiber 12 and the SAP constituting the first absorbent body 4 are mixed and supplied, whereby the first absorbent body 4 is mixed into the second mixed fiber 12.

Note that two SAP-absent regions 131 are provided on the first absorbent body 4, and the number of the SAP-absent regions may be one or three or more. Note that the basis weight of the first absorbent body 4 is in a range of 130 g/m² to 170 g/m², which is larger than the basis weight of the SAP of the second absorbent body 6.

The third mixed fiber 13 has a basis weight of 10 g/m² to 20 g/m², and contains a mixture of the crushed pulp in a proportion of 0% to 15% and the thermally-fusible fiber in a proportion of 85% to 100%.

As described above, also in the present embodiment, the SAP region can be formed separately like the second absorbent body 6, and the SAP region can be formed so as to be mixed into the fiber layer, like the first absorbent body 4. Note that, when the SAP region is formed separately like the second absorbent body 6, it can be said that the SAP region is an independent region even when a certain number of fibers is mixed into the SAP region.

In the second nonwoven fabric 14, a spunbonded nonwoven fabric having a basis weight of 8 g/m² to 12 g/m² is used, and other nonwoven fabrics can also be used.

In the present embodiment, the basis weights of the SAP of the first absorbent body 4 and the SAP of the second absorbent body 6 are approximately 235 g/m², whereas the basis weights of the first mixed fiber 11, the second mixed fiber 12, the third mixed fiber 13, and the second nonwoven fabric 14 are approximately 95 g/m². The basis weights of the SAP of the first absorbent body 4 and the SAP of the second absorbent body 6 are 2 times or more (2.4 times or more), i.e., less than 3 times the above basis weights.

Fig. 24 is a diagram illustrating a production line 600 of the multi-ply sheet 25, and the same configurations as the configurations of the production lines 100, 200, 300, 400, and 500 are referred to by the same reference signs, and the descriptions thereof are omitted or simplified. Note that, to simplify the drawing, the oscillator 135 and the masking unit 140 provided in the SAP supply device 120 are not illustrated.

The crushed pulp obtained by defibrating the pulp raw fabric 101 by the defibration device 102 and the thermally-fusible fiber from the thermally-fusible fiber supply device 107 are supplied to the conveyance belt 104, and the first mixed fiber 11 is formed.

The SAP from the SAP supply device 120 separated from the fiber stacking unit is supplied to the first mixed fiber 11, and the second absorbent body 6 is formed.

Note that a part of the SAP from the SAP supply device 120 may be mixed with the first mixed fiber 11 and the second mixed fiber 12, but in the present embodiment, even in such a case, the second absorbent body 6 is separated from the first mixed fiber 11 and the second mixed fiber 12. The layers of SAP are configured separately like the second absorbent body 6, it is possible to make the absorbent article 1 soft to the touch when the SAP absorbs body fluids such as urine.

The crushed pulp obtained by defibrating the pulp raw fabric 108 by the defibration device 102 and the thermally-fusible fiber from the thermally-fusible fiber supply device 107, in a state mixed with the SAP from the SAP supply device 120 incorporated to the fiber stacking unit, are supplied onto the second absorbent body 6. Thus, a layer in which the SAP of the first absorbent body 4 is mixed is formed on the second mixed fiber 12. The SAP is thus mixed into the fiber layer, whereby the layer structure of the absorbent article 1 can be reduced and the absorbent article 1 can be made thinner.

After that, the crushed pulp obtained by defibrating the pulp raw fabric 128 by the defibration device 102 and the thermally-fusible fiber from the thermally-fusible fiber supply device 107 are supplied onto the second mixed fiber 12, and the third mixed fiber 13 is formed.

The second nonwoven fabric 14 is supplied from the second nonwoven raw fabric 129 onto the third mixed fiber 13, and pressed and heat-treated to produce the multi-ply sheet 25. Further, the joint portions 130 can be produced by the heating device 112, the pair of embossing rollers 113, and the like.

Note that, in the production line 600, respective layers are layered sequentially from the first mixed fiber 11 side to layer the multi-ply sheet 25, but respective layers may be layered sequentially from the second nonwoven fabric 14 side to form the multi-ply sheet 25. Further, as described in the sixth embodiment, the fiber stacking unit including the defibration device 102 and the thermally-fusible fiber supply device 107 may be provided with a member that masks the supply of the crushed pulp and the thermally-fusible fiber, like the masking unit 140. In this case, the supply of the crushed pulp and the thermally-fusible fiber may be partially blocked or totally blocked. For example, the supply of the crushed pulp and the thermally-fusible fiber may be totally blocked to form the SAP layers separately. An absorbent article is produced by the above method, so that it is possible to join and integrate respective layers without using a hot-melt adhesive or the like, the porous portion of the SAP is less covered with the thermally-fusible fiber 5 compared to the hot melt, and thus there is less risk of degrading the absorption performance of SAP. Therefore, it is possible to obtain an absorbent article having excellent absorbency of body fluids and a thin sheet thickness and excellent absorbency of body fluids, as compared to the absorbent article obtained by joining and integrating respective layers by the hot melt.

### Eighth Embodiment

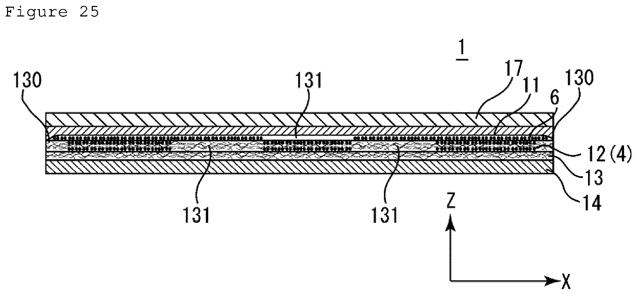

An eighth embodiment is described below, and the same configurations as the configurations in the first to seventh embodiments are referred to by the same reference signs, and the descriptions thereof are omitted or simplified. Fig. 25 is a cross-sectional view of the absorbent article 1 of the eighth embodiment, and the description is continued hereinbelow with reference to Fig. 25. The absorbent article 1 of the eighth embodiment is a multi-ply sheet 30 in which the air-through nonwoven fabric 17, the first mixed fiber 11, the second absorbent body 6, the second mixed fiber 12, the third mixed fiber 13, and the second nonwoven fabric 14 are layered in this order from above. Note that, in the present embodiment, the first absorbent body 4 is mixed into the second mixed fiber 12.

The basis weight of the air-through nonwoven fabric 17 is in a range of 10 g/m² to 40 g/m², and preferably in a range of 20 g/m² to 30 g/m².

The first mixed fiber 11 has a basis weight of 8 g/m² to 25 g/m², and contains a mixture of the crushed pulp in a proportion of 0% to 20% and the thermally-fusible fiber in a proportion of 80% to 100%.

The basis weight of the SAP of the second absorbent body 6 is in a range of 50 g/m² to 100 g/m², and preferably in a range of 60 g/m² to 80 g/m². Further, in the present embodiment, the SAP-absent regions 131 are formed in the central portion. The width of the SAP-absent regions 131 is, for example, in a range of 8 mm to 16 mm. Note that the first mixed fiber 11 is mixed into the SAP-absent region 131 in some cases.

The second mixed fiber 12 has a basis weight of 30 g/m² to 60 g/m², and contains a mixture of the crushed pulp in a proportion of 70% to 90% and the thermally-fusible fiber in a proportion of 10% to 30%.

The basis weight of the first absorbent body 4 is in a range of 180 g/m² to 250 g/m², which is larger than the basis weight of the SAP of the second absorbent body 6. Further, two (plural) SAP-absent regions 131 are formed in the first absorbent body 4, and the width of the SAP-absent regions 131 is, for example, in a range of 8 mm to 16 mm.

The third mixed fiber 13 has a basis weight of 10 g/m² to 20 g/m², and contains a mixture of the crushed pulp in a proportion of 0% to 15% and the thermally-fusible fiber in a proportion of 85% to 100%.

In the second nonwoven fabric 14, a spunbonded nonwoven fabric having a basis weight of 8 g/m² to 12 g/m² is used, and other nonwoven fabrics can also be used. Note that, in the present embodiment, the second nonwoven fabric 14 is a hydrophobic nonwoven fabric.

In the present embodiment, the basis weights of the SAP of the first absorbent body 4 and the SAP of the second absorbent body 6 are approximately 300 g/m², whereas the basis weights of the air-through nonwoven fabric 17, the first mixed fiber 11, the second mixed fiber 12, the third mixed fiber 13, and the second nonwoven fabric 14 are approximately 115 g/m². The basis weights of the SAP of the first absorbent body 4 and the SAP of the second absorbent body 6 are 2 times or more (2.6 times or more), i.e., less than 3 times the above basis weights.

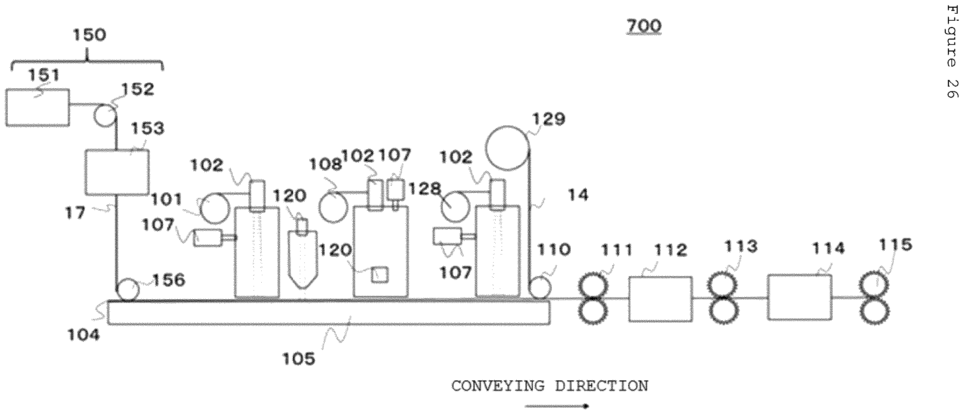

Fig. 26 is a diagram illustrating a production line 700 of the multi-ply sheet 30, and the same configurations as the configurations of the production lines 100, 200, 300, 400, 500, and 600 are referred to by the same reference signs, and the descriptions thereof are omitted or simplified. Note that, to simplify the drawing, the oscillator 135 and the masking unit 140 provided in the SAP supply device 120 are not illustrated.

The air-through nonwoven fabric 17 produced by the air-through non-woven fabric production apparatus 150 is supplied to the conveyance belt 104. Note that the air-through nonwoven fabric 17 may be embossed, or need not be embossed.

The crushed pulp obtained by defibrating the pulp raw fabric 101 by the defibration device 102 and the thermally-fusible fiber from the thermally-fusible fiber supply device 107 are supplied to the air-through nonwoven fabric 17, and the first mixed fiber 11 is formed.

The SAP from the SAP supply device 120 separated from the fiber stacking unit is supplied to the first mixed fiber 11, and the second absorbent body 6 is formed.

The crushed pulp obtained by defibrating the pulp raw fabric 108 by the defibration device 102 and the thermally-fusible fiber from the thermally-fusible fiber supply device 107, in a state mixed with the SAP from the SAP supply device 120 incorporated to the fiber stacking unit, are supplied onto the second absorbent body 6. Thus, a layer in which the SAP of the first absorbent body 4 is mixed is formed on the second mixed fiber 12.

After that, the crushed pulp obtained by defibrating the pulp raw fabric 128 by the defibration device 102 and the thermally-fusible fiber from the thermally-fusible fiber supply device 107 are supplied onto the second mixed fiber 12, and the third mixed fiber 13 is formed.

The second nonwoven fabric 14 is supplied from the second nonwoven raw fabric 129 onto the third mixed fiber 13, and pressed and heat-treated to produce the multi-ply sheet 25. Further, the joint portions 130 can be produced by the heating device 112, the pair of embossing rollers 113, and the like.

Note that, in the production line 700, respective layers are layered sequentially from the air-through nonwoven fabric 17 side to form the multi-ply sheet 30, but respective layers may be layered sequentially from the second nonwoven fabric 14 side to form the multi-ply sheet 30.

In the seventh and eighth embodiments as well, similarly to the sixth embodiment, the layer of the SAP of the second absorbent body 6 is configured separately from the other layers, and thus it is possible to make the absorbent article 1 soft to the touch when the SAP absorbs body fluids such as urine. Further, since the first absorbent body 4 is mixed into the second mixed fiber 12, when body fluids such as urine is absorbed, the texture becomes rigid to the touch, compared to the texture of the second absorbent body 6. As described above, also in the seventh and eighth embodiments, the rigidity and softness of the absorbent article 1 can be adjusted. An absorbent article is produced by the above method, so that it is possible to join and integrate respective layers without using a hot-melt adhesive or the like, the porous portion of the SAP is less covered with the thermally-fusible fiber 5 compared to the hot melt, and thus there is less risk of degrading the absorption performance of SAP. Therefore, it is possible to obtain an absorbent article having excellent absorbency of body fluids and a thin sheet thickness and excellent absorbency of body fluids, as compared to the absorbent article obtained by joining and integrating respective layers by the hot melt.

### Ninth Embodiment

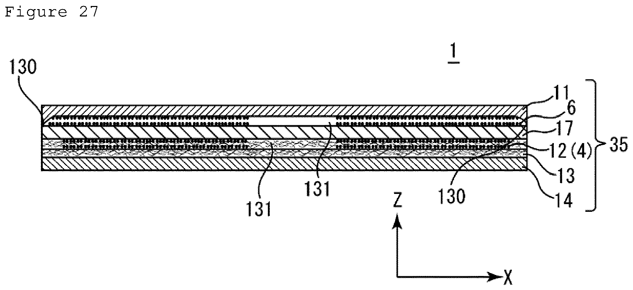

A ninth embodiment is described below, and the same configurations as the configurations in the first to eighth embodiments are referred to by the same reference signs, and the descriptions thereof are omitted or simplified. Fig. 27 is a cross-sectional view of the absorbent article 1 of the ninth embodiment, and the description is continued hereinbelow with reference to Fig. 27. The absorbent article 1 of the ninth embodiment is a multi-ply sheet 35 in which the first mixed fiber 11, the second absorbent body 6, the air-through nonwoven fabric 17, the second mixed fiber 12, the third mixed fiber 13, and the second nonwoven fabric 14 are layered in this order from above. Note that, in the present embodiment, the first absorbent body 4 is mixed into the second mixed fiber 12.

The first mixed fiber 11 has a basis weight of 8 g/m² to 25 g/m², and contains a mixture of the crushed pulp in a proportion of 0% to 20% and the thermally-fusible fiber in a proportion of 80% to 100%.

The basis weight of the SAP of the second absorbent body 6 is in a range of 180 g/m² to 300 g/m², and preferably in a range of 200 g/m² to 240 g/m². Further, in the present embodiment, the SAP-absent regions 131 are formed in the central portion. The width of the SAP-absent regions 131 is, for example, in a range of 8 mm to 16 mm. Note that the first mixed fiber 11 is mixed into the SAP-absent region 131 in some cases.

The basis weight of the air-through nonwoven fabric 17 is in a range of 10 g/m² to 40 g/m², and preferably in a range of 20 g/m² to 30 g/m².

The second mixed fiber 12 has a basis weight of 30 g/m² to 60 g/m², and contains a mixture of the crushed pulp in a proportion of 70% to 90% and the thermally-fusible fiber in a proportion of 10% to 30%.

Note that the basis weight of the first absorbent body 4 is in a range of 160 g/m² to 240 g/m², which is smaller than the basis weight of the SAP of the second absorbent body 6. Further, the SAP-absent region 131 is formed in the central portion of the first absorbent body 4, similarly to the second absorbent body 6. The width of the SAP-absent region 131 of the first absorbent body 4 is also identical to the width of the SAP-absent region 131 of the second absorbent body 6.

The third mixed fiber 13 has a basis weight of 10 g/m² to 20 g/m², and contains a mixture of the crushed pulp in a proportion of 15% to 30% and the thermally-fusible fiber in a proportion of 70% to 85%.

In the second nonwoven fabric 14, a spunbonded nonwoven fabric having a basis weight of 8 g/m² to 12 g/m² is used, and other nonwoven fabrics can also be used. In the present embodiment, the second nonwoven fabric 14 may be a hydrophobic nonwoven fabric or a hydrophilic nonwoven fabric.

In the present embodiment, the basis weights of the SAP of the first absorbent body 4 and the SAP of the second absorbent body 6 are approximately 390 g/m², whereas the basis weights of the first mixed fiber 11, the second mixed fiber 12, the air-through nonwoven fabric 17, the third mixed fiber 13, and the second nonwoven fabric 14 are approximately 125 g/m². The basis weights of the SAP of the first absorbent body 4 and the SAP of the second absorbent body 6 are 3 times or more, i.e., less than 4 times the above basis weights. As described above, in the present embodiment, since a large amount of the SAP is used, and the basis weight of the SAP is approximately 390 g/m², so that a large amount of body fluids can be absorbed.

As described above, the air-through nonwoven fabric 17 contains the thermally-fusible fiber, and the thermally-fusible fiber do not retain body fluids. In the present embodiment, the first absorbent body 4 and the second absorbent body 6 are provided with the air-through nonwoven fabric 17 interposed therebetween. Therefore, the absorbent article 1 of the present embodiment can improve the absorption speed of the body fluids.

Further, since the proportion of the thermally-fusible fiber of the first mixed fiber 11 at the skin surface side is high, the fibers fused by heating of the heating devices 112 and 114 retain the layered state of the multi-ply sheet 35. Consequently, the multi-ply sheet 35 is not wound.

### Tenth Embodiment

In a tenth embodiment, as will be described, the configuration of the tenth embodiment is the same as the configuration of the ninth embodiment. Accordingly, the description will focus on the difference in the basis weight of the multi-ply sheet 35.

The first mixed fiber 11 has a basis weight of 8 g/m² to 25 g/m², and contains a mixture of the crushed pulp in a proportion of 40% to 60% and the thermally-fusible fiber in a proportion of 40% to 60%.

The basis weight of the SAP of the second absorbent body 6 is in a range of 150 g/m² to 240 g/m², and preferably in a range of 160 g/m² to 180 g/m².

The basis weight of the air-through nonwoven fabric 17 is in a range of 10 g/m² to 40 g/m², and preferably in a range of 20 g/m² to 30 g/m².

The second mixed fiber 12 has a basis weight of 30 g/m² to 60 g/m², and contains a mixture of the crushed pulp in a proportion of 70% to 90% and the thermally-fusible fiber in a proportion of 10% to 30%.

Note that the basis weight of the first absorbent body 4 is in a range of 80 g/m² to 120 g/m², which is smaller than the basis weight of the SAP of the second absorbent body 6.

The third mixed fiber 13 has a basis weight of 10 g/m² to 20 g/m², and contains a mixture of the crushed pulp in a proportion of 15% to 30% and the thermally-fusible fiber in a proportion of 70% to 85%.

In the second nonwoven fabric 14, a spunbonded nonwoven fabric having a basis weight of 8 g/m² to 12 g/m² is used, and other nonwoven fabrics can also be used. In the present embodiment, the second nonwoven fabric 14 may be a hydrophobic nonwoven fabric or a hydrophilic nonwoven fabric.

In the present embodiment, the basis weights of the SAP of the first absorbent body 4 and the SAP of the second absorbent body 6 are approximately 270 g/m², whereas the basis weights of the first mixed fiber 11, the second mixed fiber 12, the air-through nonwoven fabric 17, the third mixed fiber 13, and the second nonwoven fabric 14 are approximately 110 g/m². The basis weights of the SAP of the first absorbent body 4 and the SAP of the second absorbent body 6 are 2 times or more, i.e., less than 3 times the above basis weights. As described above, in the present embodiment, the basis weight of the SAP and the total of the basis weights of the first mixed fiber 11, the second mixed fiber 12, the air-through nonwoven fabric 17, the third mixed fiber 13, and the second nonwoven fabric 14 are lower than those in the ninth embodiment. Accordingly, it is possible to realize the absorbent article 1 which is inexpensive.

Also, in the present embodiment, the first absorbent body 4 and the second absorbent body 6 are provided with the air-through nonwoven fabric 17 interposed therebetween. Therefore, the absorbent article 1 of the present embodiment can improve the absorption speed of the body fluids.

Further, since the proportion of the thermally-fusible fiber of the first mixed fiber 11 at the skin surface side is in a range of 40% to 60%, the fibers fused by heating of the heating devices 112 and 114 retain the layered state of the multi-ply sheet 35. Consequently, the multi-ply sheet 35 is not wound.

In the ninth and tenth embodiments as well, the layer of the SAP of the second absorbent body 6 is configured separately from the other layers, and thus it is possible to make the absorbent article 1 soft to the touch when the SAP absorbs body fluids such as urine. Further, since the first absorbent body 4 is mixed into the second mixed fiber 12, when body fluids such as urine is absorbed, the texture becomes rigid to the touch, compared to the texture of the second absorbent body 6. As described above, also in the seventh and eighth embodiments, the rigidity and softness of the absorbent article 1 can be adjusted.

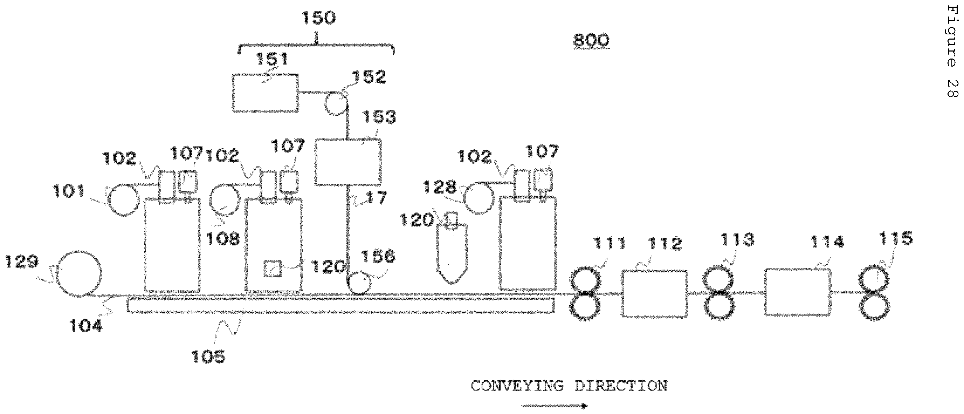

The method for producing the absorbent article 1 of the ninth and tenth embodiments is described below. Fig. 28 is a diagram illustrating a production line 800 of the multi-ply sheet 30, and the same configurations as the configurations of the production lines 100, 200, 300, 400, 500, 600, and 700 are referred to by the same reference signs, and the descriptions thereof are omitted or simplified. Note that, in the production line 800, the second nonwoven fabric 14 is first supplied to the conveyance belt, and then the components are sequentially supplied onto the second nonwoven fabric 14 to produce the multi-ply sheet 35, however the present invention is not limited thereto.

The second nonwoven fabric 14 (not illustrated in Fig. 28) from the second nonwoven raw fabric 129 is supplied to the conveyance belt 104. The crushed pulp obtained by defibrating the pulp raw fabric 101 by the defibration device 102 and the thermally-fusible fiber from the thermally-fusible fiber supply device 107 are supplied to the second nonwoven fabric 14, and the third mixed fiber 13 is formed.

The crushed pulp obtained by defibrating the pulp raw fabric 108 by the defibration device 102 and the thermally-fusible fiber from the thermally-fusible fiber supply device 107, in a state mixed with the SAP from the SAP supply device 120 incorporated to the fiber stacking unit, are supplied onto the third mixed fiber 13. Thus, a layer in which the SAP of the first absorbent body 4 is mixed is formed on the second mixed fiber 12.

The air-through nonwoven fabric 17 produced by the air-through non-woven fabric production apparatus 150 is supplied to the second mixed fiber 12. Note that the air-through nonwoven fabric 17 may be embossed, or need not be embossed. Note that it is also possible to use the raw fabric of the air-through nonwoven fabric 17 produced at a place different from the production line 800 without the air-through non-woven fabric production apparatus 150 in-line.

Then, the SAP from the SAP supply device 120 is supplied onto the air-through nonwoven fabric 17, and the second absorbent body 6 separated from the other layers is formed.

Thereafter, the crushed pulp obtained by defibrating the pulp raw fabric 128 by the defibration device 102 and the thermally-fusible fiber from the thermally-fusible fiber supply device 107 are supplied onto the second absorbent body 6, and the first mixed fiber 11 is formed.

Further, heating is performed by the heating device 112 and the heating device 114 in a similar manner to the production lines 100 to 700 described above. In the production line 800, the amount of heat given to the multi-ply sheet 35 by the heating device 112 is larger than the amount of heat given to the multi-ply sheet 35 by the heating device 114. In this case, the length of the heating device 112 may be set to a length longer than the length of the heating device 114 to lengthen the time for heating the multi-ply sheet 35, and the heating time may be set to the same as the heating time to increase the amount of heat given by the heating device 112.

Further, the heating device 112 heats the multi-ply sheet 35 from the upper side (side of the first mixed fiber 11) and the heating device 114 heats the multi-ply sheet 35 from the lower side (side of the second nonwoven fabric 14), so that it is possible to apply a balanced amount of heat to the multi-ply sheet 35. Such heating by the heating devices 112 and 114 can also be applied to the production lines 100 to 700 as described above. An absorbent article is produced by the above method, so that it is possible to join and integrate respective layers without using a hot-melt adhesive or the like. The porous portion of the SAP is less covered with the thermally-fusible fiber 5 compared to the hot melt, and thus there is less risk of degrading the absorption performance of SAP. Then, the absorbent article having excellent absorbency of body fluids and a thin sheet thickness and excellent absorbency of body fluids, as compared to the absorbent article obtained by joining and integrating respective layers by the hot melt.

### Examples

There is described an example in which the multi-ply sheet 15 having the configuration illustrated in Fig. 11 is used as the absorbent article 1. In this example, each of the first nonwoven fabric 10 and the second nonwoven fabric 14 is a spunbonded nonwoven fabric having a basis weight of 15 g/m².

The first mixed fiber 11 has a basis weight of 10 g/m², and contains a mixture of the thermally-fusible fiber in a proportion of 95% and the crushed pulp in a proportion of 5%. The basis weight of the SAP for the first absorbent body 4 and the SAP of the second absorbent body are each 10 g/m². The basis weight of the second mixed fiber 12 and the basis weight of the third mixed fiber 13 are each 50 g/m², and contain a mixture of the thermally-fusible fiber in a proportion of 20% and the crushed pulp in a proportion of 80%. Further, two SAP-absent regions 131 were formed in each absorption layer. Five absorbent articles 1 having a size of 100 mm in length and 360 mm in width were prepared.

### (Absorption Rate Test)

There is prepared a water injection jig in which an acrylic pipe with an inner diameter of about 25 mm and a height of about 200 mm is inserted into the center of a square base of about 100 mm x 100 mm and about 1 kg. This water injection jig is set approximately in the center of the absorbent article 1 (may be shifted in the longitudinal direction by several tens of mm from the center).

80 cc of 0.9% physiological saline is prepared, the physiological saline is supplied from the water injection jig to the absorbent article 1 at an almost constant speed (range of 80 cc/12 seconds to 80 cc/10 seconds). The time from the start of supply of physiological saline to the end of absorption of physiological saline (the time until the physiological saline disappears from the surface of the absorbent article 1) (first absorption time) is measured. After 1-minute interval, 80 cc of 0.9% physiological saline is prepared, and the time required for the second absorption (second absorption time) is measured in the above-mentioned manner.

When the absorption rate was measured using the five absorbent articles 1, the first absorption times were 26 seconds, 28 seconds, 24 seconds, 26 seconds, and 29 seconds, all of which were in a range of 24 seconds to 30 seconds. The average of the first absorption times was 26.6 seconds. Further, the second absorption times were 17 seconds, 16 seconds, 17 seconds, 19 seconds, and 18 seconds, all of which were in the range of 16 to 20 seconds, and the average of the second absorption times was 17.4 seconds.

### (Rewet Amount Test)

5 minutes after the second absorption of physiological saline, the water injection jig is removed, and then weighed filter paper (diameter: 9 cm) is placed on the absorbent article 1. A weight (about 2 kg) is placed on the filter paper so that a load of about 30 g/cm² is applied to the filter paper. 1 minute after the weight was placed, the weight of the filter paper was measured, and the amount increased with respect to the weight of the filter paper before being placed on the absorbent article 1 was defined as the rewet amount. The rewet amounts were 0.14 g, 0.09 g, 0.08 g, 0.12 g, and 0.09 g, all of which were in the range of 0.08 g to 0.14 g, and the average of the rewet amounts was 0.1 g.

In this example, the side of the absorption of physiological saline is continued to the first nonwoven fabric 10, and the first mixed fiber 11 having a basis weight of 10 g/m² and containing a mixture of the thermally-fusible fiber in a proportion of 95% and the crushed pulp in a proportion of 5% is provided. Since the thermally-fusible fiber does not substantially absorb the physiological saline, the physiological saline transfers to the second absorbent body 6. The second absorbent body 6 absorbs the physiological saline in the first absorption and the physiological saline in the second absorption and swells, and transfers the physiological saline unabsorbed by the second absorbent body 6 to the first absorbent body 4 through the second mixed fiber 12. Thus, the physiological saline hardly returns to the side of the first nonwoven fabric 10. The proportion of the thermally-fusible fiber is increased at the water absorption side, the proportion of the thermally-fusible fiber is decreased toward the lower layer side, and the amount of the crushed pulp is increased, whereby the physiological saline is absorbed by the first absorbent body 4 while the physiological saline is retained by the crushed pulp. Further, the SAP-absent regions 131 are provided over the two layers, whereby the possibility that the gel blocking phenomenon occurs is reduced.

Note that, in the above-mentioned examples, the mixing rate of the first mixed fiber 11 is desirably in a range of 80% to 100%. Further, the amount of the SAP in the first absorbent body 4 and the amount of the SAP in the second absorbent body are not necessarily identical. The basis weight of the second mixed fiber 12 and the basis weight of the third mixed fiber 13 may be different, and the mixing rate of the thermally-fusible fiber and the crushed pulp may be different.

Note that the present invention is not limited to the above-mentioned embodiments, and each of the embodiments can be combined or modified as appropriate. For example, the stacking order of the sheet layers of the absorbent article 1 may be changed, or any one of the sheet layers of the absorbent article 1 may be omitted. Further, bead-shaped polyethylene powder may be used instead of the thermally-fusible fiber.

Note that, in the present invention, as a unit for forming regions (groove portions, or the like) without the first absorbent body 4 and the second absorbent body 6, any unit usually used for forming groove portions or the like, such as pressing by embossing, ultrasonic bonding, heat-sealing, or the like, can be preferably used, in addition to the units described in the above-mentioned embodiments.

Further, in the present invention, when notch portions (slit portions) are formed as the region without the first absorbent body 4 and the region without the second absorbent body 6, it is also preferable to perform a pressing process along the slit portions in order to prevent the SAP or the fiber from falling off the notch portions (slit portions).

Note that, in the multi-ply sheet of the present invention, it is also preferable to provide various types of layers (paper, nonwoven fabric, porous film, and the like) as intermediate layers. Such intermediate layers are provided, so that the performance of the multi-ply sheet can be further improved from various viewpoints. For example, when a material containing pulp as a main raw material such as paper is provided as an intermediate layer, the absorption function of the multi-ply sheet may be further improved due to its high liquid diffusibility.

Further, ultrasonic bonding may be performed in order to strengthen the joint of the joint portion 130 formed by thermal fusion.

Furthermore, the heating device 114 may be omitted, or the protrusion portion 18 and the recess portion 19 illustrated in Fig. 18 may be omitted.

### Reference Signs List

- 1: Absorbent article
- 2: First liquid-diffusible fiber sheet
- 3: First pulp layer
- 4: First absorbent body
- 5: Thermally-fusible fiber
- 6: Second absorbent body
- 7: Second pulp layer
- 8: Second liquid-diffusible fiber sheet
- 9: Multi-ply sheet

## Claims

1. An absorbent article comprising:
a first absorbent body configured to absorb body fluids;
a second absorbent body configured to absorb body fluids; and
a thermally-fusible fiber provided between the first absorbent body and the second absorbent body and capable of retaining the first absorbent body and the second absorbent body.

2. The absorbent article according to claim 1, wherein at least one of the first absorbent body or the second absorbent body has a super absorbent polymer.

3. The absorbent article according to claim 1, wherein at least one of the first absorbent body or the second absorbent body is formed by fusing and fixing a super absorbent polymer with a hot-melt fiber.

4. The absorbent article according to claim 1, wherein the first absorbent body is provided at a non-skin surface side with respect to the second absorbent body, and
includes a cover sheet having air permeability and covering the first absorbent body.

5. The absorbent article according to claim 1, wherein the second absorbent body is provided at a skin surface side with respect to the first absorbent body, and
includes:
a cover sheet covering the second absorbent body; and
a flexible layer provided between the second absorbent body and the cover sheet and having flexibility.

6. The absorbent article according to claim 1,
wherein the first absorbent body is provided at a non-skin surface side with respect to the second absorbent body, and
includes a first cover sheet covering the first absorbent body,
a second cover sheet covering the second absorbent body provided at a skin surface side with respect to the first absorbent body, and
a first flexible layer provided between the first absorbent body and the first cover sheet and having flexibility, and a second flexible layer provided between the second absorbent body and the second cover sheet and having flexibility.

7. The absorbent article according to claim 1, wherein the first absorbent body is provided at a non-skin surface side with respect to the second absorbent body, the first absorbent body and the second absorbent body each have a super absorbent polymer, and the super absorbent polymer of the second absorbent body has an average particle size larger than an average particle size of the super absorbent polymer of the first absorbent body.

8. The absorbent article according to claim 6, wherein a diffusion rate of liquids of the first cover sheet and the second cover sheet is faster than a diffusion rate of liquids of the first flexible layer and the second flexible layer.

9. An absorbent article comprising:
an absorbent body configured to absorb body fluids;
a thermally-fusible fiber capable of retaining the absorbent body;
a cover sheet covering the absorbent body; and
a flexible layer provided between the absorbent body and the cover sheet and having flexibility.

10. The absorbent article according to claim 9, wherein a super absorbent polymer is retained in the absorbent body by melting the thermally-fusible fiber.

11. The absorbent article according to claim 9, wherein the flexible layer has crushed pulp.

12. The absorbent article according to claim 9, wherein the cover sheet is embossed.

13. An absorbent article comprising:
a first fiber having a thermally-fusible fiber;
a second fiber having the thermally-fusible fiber and crushed pulp, the crushed pulp having a higher proportion than the thermally-fusible fiber;
a third fiber having the thermally-fusible fiber and the crushed pulp, the crushed pulp having a higher proportion than the thermally-fusible fiber;
a first absorbent body provided between the second fiber and the third fiber; and
a second absorbent body provided between the first fiber and the second fiber.

14. The absorbent article according to claim 13, wherein the first fiber has the thermally-fusible fiber and the crushed pulp, and the thermally-fusible fiber has a higher proportion than the crushed pulp.

15. The absorbent article according to claim 13, wherein at least one of the first absorbent body or the second absorbent body has a region in which an absorbent body is partially absent and a region in which the absorbent body is present.

16. An absorbent article comprising:
a first layer having a thermally-fusible fiber or a nonwoven fabric;
a second layer having the thermally-fusible fiber or the nonwoven fabric;
a first region in which an absorbent body is provided between the first layer and the second layer; and
a second region in which the absorbent body is not provided between the first layer and the second layer,
wherein the first layer and the second layer in the second region are melted and joined.

17. The absorbent article according to claim 16, further comprising an air-through nonwoven fabric provided between the first layer and the second layer and capable of retaining the absorbent body.

18. The absorbent article according to claim 17, wherein a recess portion and a protrusion portion are formed in the air-through nonwoven fabric, and a liquid diffusibility of the recess portion is higher than a liquid diffusibility of the protrusion portion.

19. The absorbent article according to claim 16, wherein the absorbent body is provided in a state mixed with a fiber.

20. An absorbent article comprising:
a first layer;
a second layer;
a first absorbent body provided between the first layer and the second layer, and provided in a state mixed with a fiber;
a second absorbent body provided between the first layer and the second layer, and provided separately from the fiber.

21. The absorbent article according to claim 20, wherein the second absorbent body is provided at a skin surface side with respect to the first absorbent body.

22. The absorbent article according to claim 20, wherein the first absorbent body provided in the state mixed with the fiber includes a first region in which an absorbent body is provided and a second region in which the absorbent body is not provided.

23. An absorbent article in which a flexible crushed pulp layer containing a thermally-fusible resin and an absorbent body layer containing a super absorbent polymer, that are sequentially layered by flowing air, are joined to each other by heating with hot air and integrated.

24. An absorbent article in which layers on sides of at least one end and an opposing end of an absorbent body have a portion where a super absorbent polymer is substantially absent, and, in the portion, a flexible layer and an absorbent body layer are joined by heating with hot air and integrated.

25. The absorbent article according to claim 24, wherein a joint portion between the flexible layer and the layer of the absorbent body is pressed before and/or after the heat treatment with hot air.
